(19) Europäisches Patentamt
European Patent Office
Office européen des brevets

(11) EP 1 919 894 B1

(12) **FASCICULE DE BREVET EUROPEEN**

(45) Date de publication et mention de la délivrance du brevet:
**08.10.2014 Bulletin 2014/41**

(21) Numéro de dépôt: **06794293.8**

(22) Date de dépôt: **04.08.2006**

(51) Int Cl.:
*C07D 401/06* (2006.01) *C07D 401/14* (2006.01)
*C07D 401/12* (2006.01) *A61K 31/47* (2006.01)

(86) Numéro de dépôt international:
**PCT/FR2006/001906**

(87) Numéro de publication internationale:
**WO 2007/015017 (08.02.2007 Gazette 2007/06)**

(54) **NOUVEAUX DERIVES DE POLYQUINOLEINES ET LEUR UTILISATION THERAPEUTIQUE**

NEUE POLYCHINOLINDERIVATE UND IHRE THERAPEUTISCHE VERWENDUNG

NOVEL POLYQUINOLINE DERIVATIVES AND THE THERAPEUTIC USE THEREOF

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **04.08.2005 FR 0508351**

(43) Date de publication de la demande:
**14.05.2008 Bulletin 2008/20**

(60) Demande divisionnaire:
**10185850.4 / 2 289 891**

(73) Titulaire: **Palumed S.A.**
**31320 Castanet-Tolosan (FR)**

(72) Inventeurs:
- **DERAEVE, Céline**
**F-31400 Toulouse (FR)**
- **PITIE, Marguerite**
**F-31520 Ramonville (FR)**
- **BOLDRON, Christophe**
**F-31400 Toulouse (FR)**
- **MEUNIER, Bernard**
**F-31320 Castanet (FR)**

(74) Mandataire: **Domenego, Bertrand**
**Cabinet Lavoix**
**2, place d’Estienne d’Orves**
**75441 Paris Cedex 09 (FR)**

(56) Documents cités:
**WO-A-2004/007461**

- **BRENT R. STOCKWELL, JAMES S. HARDWICK, JEFFREY K. TONG, STUART L. SCHREIBER: "Chemical Genetic and Genomic Approaches Reveal a Role for Copper in specific Gene Activation" JOURNAL OF THE AMERICAN CHEMICAL SOCIETY, vol. 121, 1999, pages 10662-10663, XP002381594 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 919 894 B1

## Description

La présente invention concerne de nouveaux dérivés poly-quinoléines, leur procédé de préparation et leur utilisation en tant qu'agents thérapeutiques.

Plus précisément, les composés selon la présente invention présentent la propriété d'être des ligands de métaux et/ou de dissoudre les agrégats amyloïdes et sont particulièrement utiles dans le traitement de maladies neurodégénératives.

Différentes revues récapitulent un ensemble de données mettant en évidence que beaucoup de désordres neurodégénératifs progressifs et lents sont associés à : (i) un stress oxydant, (ii) un mauvais repliement de protéines conduisant à la formation d'agrégats, de fibrilles, de profibrilles ou de plaques, (iii) une accumulation de ces protéines, (iv) une perte de synapses, (v) une homéostasie des ions métalliques altérée, (vi) des défauts dans les axones et le transport dendritique, (vii) une mort neuronale. (E. Bossy-Wetzel et al., Nature Medecine, 2004, S2-S9 ; K. J. Barnham et al., Nature Rev. Drug Discov., 2004, 3, 205-214; M. P. Mattson, Nature, 2004, 430, 631-639; P. M. Doraiswamy et al., The Lancet Neurol., 2004, 3, 431-434).

En effet, de nombreuses études ont récemment mis en évidence le rôle déterminant des ions métalliques (cuivre, zinc, fer, aluminium, manganèse ...) dans la modification du repliement ou de l'agrégation de protéines, conduisant ainsi à des pathologies graves. Ce rôle néfaste d'interactions hors normes d'ions métalliques avec des protéines vient d'être souligné dans de nombreuses maladies neurodégénératives (par exemple : la maladie d'Alzheimer, des encéphalopathies spongiformes, la maladie de Parkinson, la maladie d'Huntington, la sclérose latérale amyotrophique, etc.) ou lors de l'évolution néfaste de certains handicaps comme dans le cas de la trisomie 21. Une ou des protéines spécifiques sont associées à chaque maladie et il a été mis en évidence que des chélateurs d'ions métalliques peuvent être actifs pour limiter leurs mauvais repliements induits par les métaux.

Dans certaines encéphalopathies comme la maladie de Kreutzfeld-Jacob et de son nouveau variant, il est maintenant admis que ces maladies sont liées à la transformation d'une protéine de type prion (PrP) en sa forme pathologique et infectieuse dite "scrapie" ($PrP^{sc}$). Les ions cuivriques sont impliqués dans cette modification conformationnelle (formation de feuillets bêta) des prions qui deviennent résistants aux protéases et insolubles dans les détergents non dénaturants. Des travaux récents viennent de montrer qu'un ligand tel que le disulfate de la bathocuproïne peut restaurer *in vitro* la sensibilité de la protéine "scrapie" $PrP^{sc}$ aux protéases et sa solubilité (E. Quaglio et al., J. Biol. Chem., 2001, 276, 11432-11438).

Dans le cas de la maladie de Parkinson, l'$\alpha$-synucléine interagit avec des ions ferriques. Il a été proposé que ces ions facilitent la formation de radicaux hydroxyles, particulièrement oxydants et des études par IRM *post mortem* ont mis en évidence de fortes concentrations d'ions ferriques dans la *substancia nigra* de malades (une zone du cerveau où les neurones dopaminergiques sont plus sélectivement touchés dans cette maladie). L'emploi de chélateurs comme le Clioquinol réduit la toxicité du 1-méthyl-4-phényl-1,2,3,6-tétrapyridine, une toxine induisant le parkinsonisme, sur les souris (D. Kaur et al., Neuron 2003, 37, 899-909).

Dans le cas de la maladie d'Alzheimer (M. P. Mattson, Nature, 2004, 430, 631-639; M. Citron, Nature Rev. Neurosci., 2004, 5, 677-685), la pathologie est liée à l'agrégation dans le cerveau de peptides $\beta$-amyloïdes conduisant à la formation des plaques amyloïdes. Cette agrégation peut être induite par les ions Cu(II) et Zn(II) mais aussi, dans une moindre mesure, Fe(III). L'accumulation au sein de ces plaques d'ions métalliques à activité rédox est probablement à l'origine du stress oxydant important (*via* la production de $H_2O_2$) lui-même à l'origine des dommages créés sur les neurones du cerveau, conduisant à une perte irréversible des facultés intellectuelles (M. P. Cuajungco et al., Ann. N. Y. Acad. Sci., 2000, 920, 292-304; C. S. Atwood et al., Met. Ions Biol. Syst., 1999, 36, 309-364). Le fait que les premiers essais effectués avec un ligand d'ions métalliques comme le Clioquinol conduisent à des améliorations dans la maladie d'Alzheimer (R. A. Cherny et al., Neuron, 2001, 30, 665-676) indiquent que des approches thérapeutiques sont possibles à l'aide de chélateurs d'ions métalliques.

Toutefois, ces chélateurs doivent posséder les propriétés suivantes pour être susceptibles d'être utilisés comme médicaments dans le traitement de maladies neurodégénératives :

- (a) être de faible masse moléculaire et ne pas être trop fortement chargés afin de pouvoir passer les différentes barrières (d'abord intestinale dans le cas d'une molécule utilisée par voie orale et ensuite, et de manière réversible, la barrière hémato-méningée pour aller chélater les ions métalliques en excès dans les protéines pathogènes),
- (b) présenter une structure modifiable afin d'ajuster la sélectivité de chélation à certains ions métalliques (une forte chélation non spécifique conduirait à une déplétion généralisée des ions métalliques, y compris ceux des métalloenzymes essentielles au fonctionnement de l'organisme) ou de permettre de moduler leur biodistribution dans l'organisme.

Des chélateurs comportant un motif quinoléine substitué en position 8 par un hétéroatome (comme les dérivés de 8-hydroxyquinoléine par exemple) sont des candidats pour chélater les excès d'ions métalliques impliqués dans les

maladies neurodégénératives. On peut s'attendre à ce que ce type de ligand forme souvent des complexes de cuivre, de zinc ou de fer (des ions métalliques associés aux agrégations protéiques et même au stress oxydant pour le cuivre et le fer) comportant deux (et même trois pour le fer) ligands autour de l'ion métallique (Sillen, L. G. et al., Stability Constants of Metal-Ion Complexes, The Chemical Society London Publication, 1971).

Des dérivés de bis-quinoléines ont été décrits, mais rarement comme agents pour le traitement de maladies du système nerveux potentiels. Il en est ainsi de WO 2004/007461 qui décrit la propriété de chélateur métallique. Néanmoins, ce document décrit essentiellement des composés mono-quinoléine. Par ailleurs, EP 0 443 862 décrit des dérivés agonistes du récepteur NMDA et ne suggère aucunement l'activité de chélateur métallique des composés décrits. Enfin, Stockwell et al. dans J. Am. Chem. Soc. 1999, 10662-10663 décrivent l'activité biologique des composés 2, 2'-(imino)bis(8-quinoléinol) et ses dérivés 2, 2'-(methylimino)- et 2, 2'-(n-butylimino)-bis(8-quinoléinol).

Il a maintenant été découvert, et ce de façon totalement inattendue, que les composés 2,2'- ou 8,8'-poly-quinoléines selon l'invention présentaient une forte activité chélatrice pour les métaux et/ou une capacité à dissoudre les agrégats amyloïdes.

Par « aggrégats amyloïdes », on entend ici une structure polymérique de peptides Aβ générée par interaction secondaire, tertiaire ou quaternaire (de feuillet β par exemple) ou par coordination biométallique sur le peptide (E. Scarpini et al., The Lancet Neurology, 2003, 2, 539-547 ; E. Gaggeli et al., Chem. Rev., 2006, 106, 1995-2044 ; A. B. Clippingdale et al., J. Peptide Sc., 2001, 7, 227-249).

Ces composés sont utiles comme médicaments pour le traitement et/ou la prévention de maladies neurodégénératives, notamment de la maladie d'Alzheimer, de Parkinson, des encéphalopathies spongiformes, la maladie d'Huntington, la sclérose latérale amyotrophique ou la trisomie 21.

Les présents inventeurs ont ainsi développé des chélateurs comportant plusieurs motifs quinoléine substitués (en position 2 ou 8) de petite taille et suffisamment hydrophobes pour pouvoir passer les barrières. Ils ont ainsi démontré que ces structures favorisaient l'interaction des hétérocycles de la molécule sur le même ion métallique et que des substitutions sur ces ligands, introduites de façon contrôlée, pouvaient moduler leur action vis-à-vis des protéines impliquées dans les maladies neurodégénératives : les propriétés de chélation en particulier des ions Cu(II), Zn(II) et Fe(III) impliquées dans ces maladies, d'hydrophobicité, de capacité à désagréger les protéines impliquées dans les maladies neurodégénératives que ce soit ou non en présence d'ion métallique, ou de diminuer le stress oxydant qu'elles peuvent induire.

La présente invention concerne les composés de formule (I)

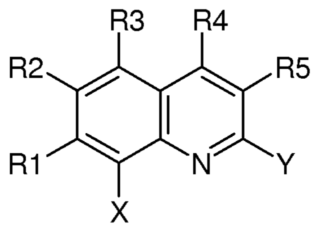

(I)

tels que dans la formule (I)
X représente un groupe -NRR', et
Y représente un groupe de formule :

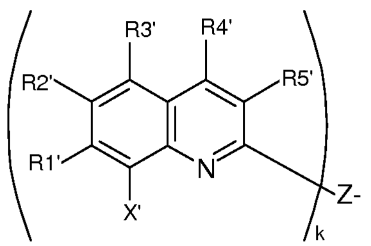

(IY)

dans laquelle X' représente un groupe -NRR', Z représente un groupe -NR''-Où R'' représente un alkyle éventuellement substitué par un ou plusieurs substituants choisis parmi OR, NRR';
R et R' identiques ou différents représentent indépendamment un atome d'hydrogène ou un groupe alkyle

R1, R2, R3, R4, R5, R1', R2', R3', R4', R5' identiques ou différents représente indépendamment un atome choisi parmi H, ou Halogène, k représente 1 ;
ainsi que leurs stéréoisomères ou leurs mélanges, leurs formes tautomères, leurs hydrates, solvates, leurs sels, formes libres et esters pharmaceutiquement acceptables.

Plus préférentiellement, les composés selon l'invention sont choisis parmi : N-Butyl-2,2'-imino-bis(8-quinoléinamine), ainsi que leurs stéréoisomères ou leur mélanges, leurs formes tautomères, leurs hydrates, solvates, leurs sels, formes libres et esters pharmaceutiquement acceptables.

Selon la présente invention, les radicaux Alkyle représentent des radicaux hydrocarbonés saturés, en chaîne droite ou ramifiée, de 1 à 20 atomes de carbone, de préférence de 1 à 5 atomes de carbone.

On peut notamment citer, lorsqu'ils sont linéaires, les radicaux méthyle, éthyle, propyle, butyle, pentyle, hexyle, octyle, nonyle, décyle, dodécyle, hexadécyle, et octadécyle.

On peut notamment citer, lorsqu'ils sont ramifiés ou substitués par un ou plusieurs radicaux alkyle, les radicaux isopropyle, tert-butyl, 2-éthylhexyle, 2-méthylbutyle, 2-méthylpentyle, 1-méthylpentyle et 3-méthylheptyle.

Parmi les atomes d'Halogène, on cite plus particulièrement les atomes de fluor, de chlore, de brome et d'iode.

Parmi les radicaux -AlkylAryle, on peut notamment citer le radical benzyle.

L'expression « sels pharmaceutiquement acceptables » fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valérate, oléate, palmitate, stéarate, laurate, borate, benzoate, lactate, phosphate, tosylate, citrate, maléate, fumarate, succinate, tartrate, naphthylate, mésylate, glucoheptanate, lacto-bionate, sulfamates, malonates, salicylates, propionates, méthylènebis-beta-hydroxynaphtoates, acide gentisique, iséthionates, di-*p*-toluoyltartrates, méthanesulfonates, éthanesulfonates, benzènesulfonates, *p*-toluènesulfonates, cyclohexyl sulfamates et quinateslaurylsulfonate, et analogues. (Voir par exemple S.M. Berge et al. Pharmaceutical Salts, J. Pharm. Sci, 66 : p.1-19 (1977) qui est incorporé ici en référence). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les aminés qui sont souvent utilisées en chimie médicinale en raison de leur faible toxicité et de leur acceptabilité pour l'usage médical : ammoniac, éthylènediamine, N-méthyl-glucamine, lysine, arginine, ornithine, choline, *N,N'*-dibenzyl-éthylenediamine, chloroprocaïne, diéthanolamine, procaïne, *N*-benzyl-phénéthylamine, diéthylamine, pipérazine, tris(hydroxyméthyl)-aminométhane, hydroxyde de tétraméthylammonium, triéthylamine, dibenzylamine, éphénamine, déhydroabiéthylamine, N-éthyl-pipéridine, benzylamine, tétraméthylammonium, tétraéthylammonium, méthylamine, diméthylamine, triméthylamine, éthylamine, acides aminés basiques, par exemple lysine et arginine, et dicyclohexylamine, et analogues.

L'invention se rapporte également aux stéréoisomères ou leurs mélanges, les formes tautomères, les hydrates, solvates, sels et esters pharmaceutiquement acceptables des composés de formule (I).

Les composés de l'invention de formule (I) définis tels que précédemment, possédant une fonction suffisamment acide ou une fonction suffisamment basique ou les deux, peuvent inclure les sels correspondants d'acide organique ou minéral ou de base organique ou minérale pharmaceutiquement acceptables.

Selon un autre objet, la présente invention concerne également le procédé de préparation des composés de formule (I).

Dans les modes de réalisation et les exemples qui vont suivre, le groupement R6 représente le groupement Y.

Les composés de formule (I) sont préparés par couplage des composés de formule (VVII) et (VVII') :

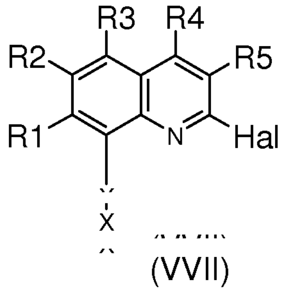

(VVII)

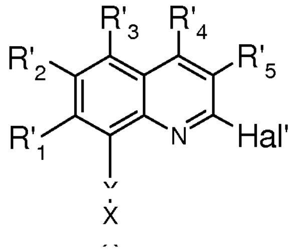

(VVII')

dans lesquelles Hal et Hal' sont des atomes d'halogène en présence d'un groupe de formule-$RNH_2$, éventuellement suivi de dérivatisation(s) du produit de formule (I) obtenu pour obtenir le produit de formule (I) souhaité.

Généralement, cette réaction est effectuée par réaction de (VVII) avec $RNH_2$ suivi du couplage avec (VVII') en présence d'un catalyseur tel que letris(dibenzylidèneacétone)dipalladium(0) et le *rac*-2,2'-bis(diphénylphosphino)-1,1',binaphthyl.

Ce procédé est illustré de façon représentative par le schéma 16.

Par « dérivatisation », on entend toute réaction permettant de modifier ou introduire des groupes sur les molécules de départ. Il peut s'agir d'halogénation, d'alkylation, d'alkoxylation, d'addition, de substitution, de protection, déprotection, de réduction, etc. Ces réactions sont généralement connues en soi. Dans le procédé selon l'invention, ces réactions peuvent être effectuées par application ou adaptation de ces méthodes.

Des exemples représentatifs de ces réactions de dérivatisation sont illustrés par les schémas 1, 3, 4, 9, 10, 13, 14, 15 et 16, ce dernier étant le seul schéma illustrant l'invention.

Ainsi, les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les exemples qui suivent.

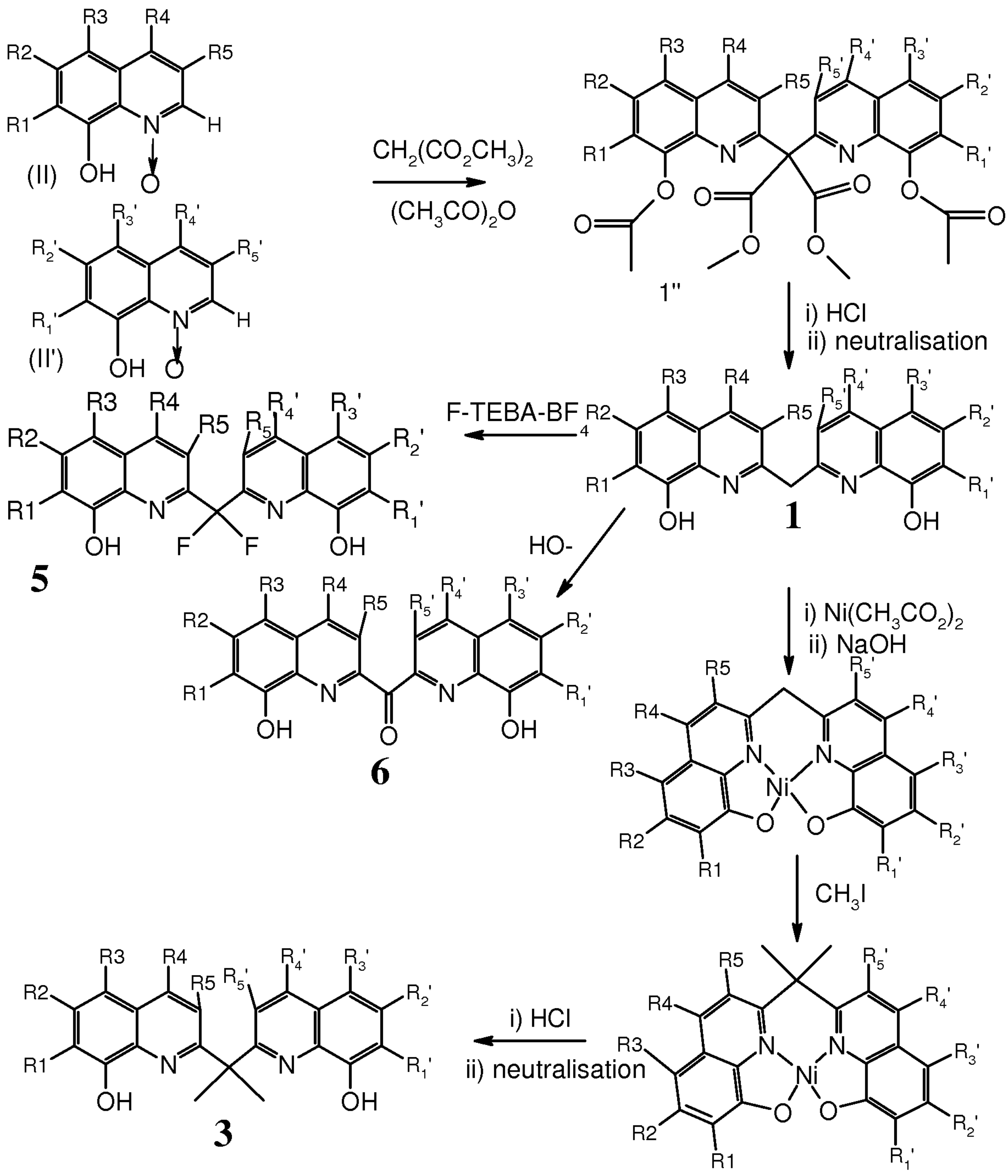


Schéma 1 : Synthèse de 1, 1'' et de leurs dérivés : **3, 5, 6**, et analogues

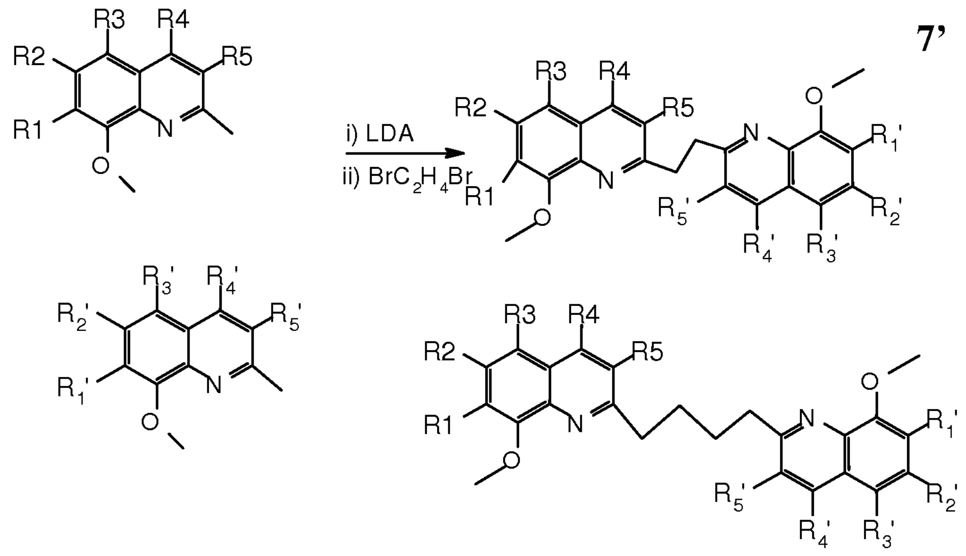


7'

10'

Schéma 2 : Synthèse de 2,2'-(1,2-éthanediyl)-bis[8-(méthyloxy)quinoléine] et de 2,2'-(1,4-butanediyl)-bis[8-(méthyloxy)quinoléine] **(7'), (10')** et leurs analogues

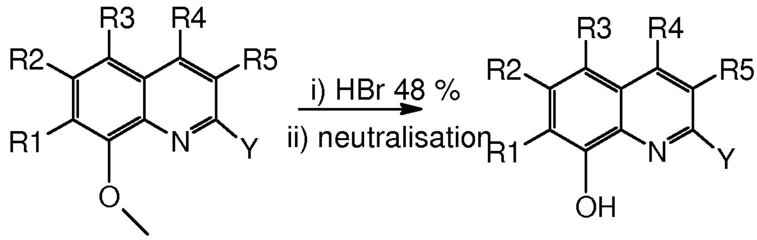


**7 10 11**

Schéma 3 : Hydrolyse des groupements protecteurs méthyloxy pour la synthèse de **7, 10, 11** et leurs analogues

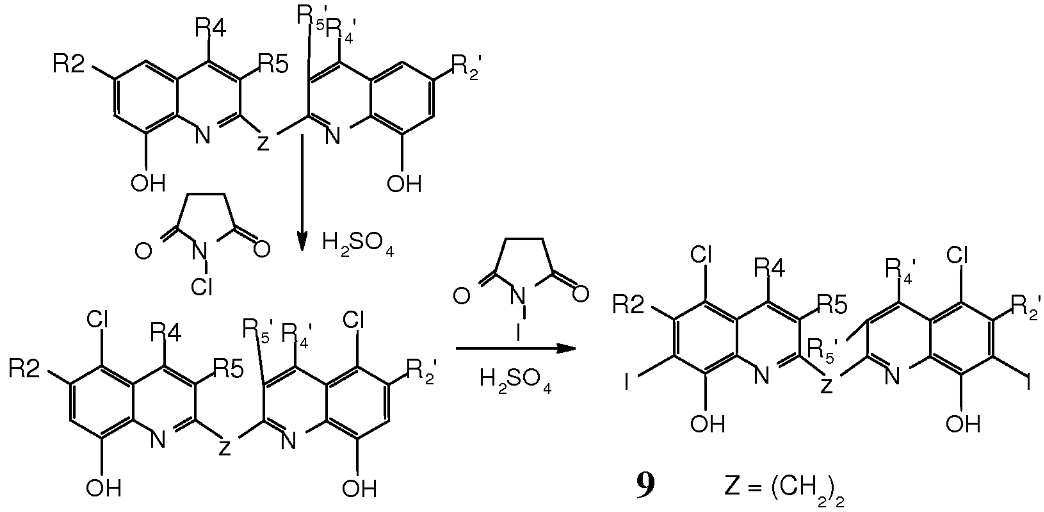


**9** Z = $(CH_2)_2$

**2** Z = $CH_2$

**4** Z = $(CH_3)_2C$

**8** Z = $(CH_2)_2$

Schéma 4 : Réaction d'halogénation pour la préparation de **2, 4, 8, 9** et leurs analogues

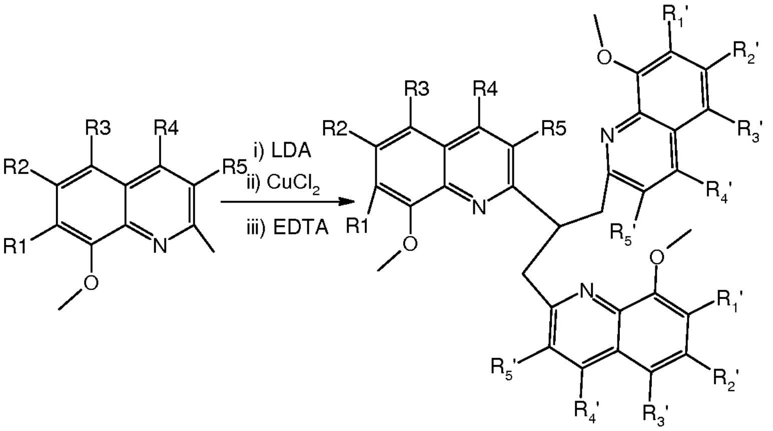


**11'**

Schéma 5 : Synthèse de 2,2',2''-(1,2,3-propanetriyl)-tris[8-(méthyloxy)quinoléine] **(11')** et leurs analogues.

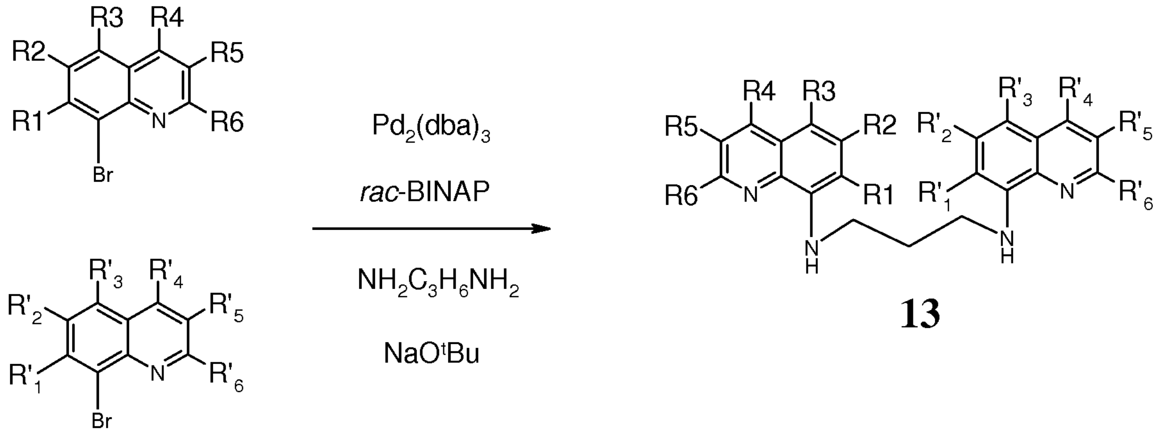


**13**

Schéma 6 : Synthèse de **13** et ses analogues.

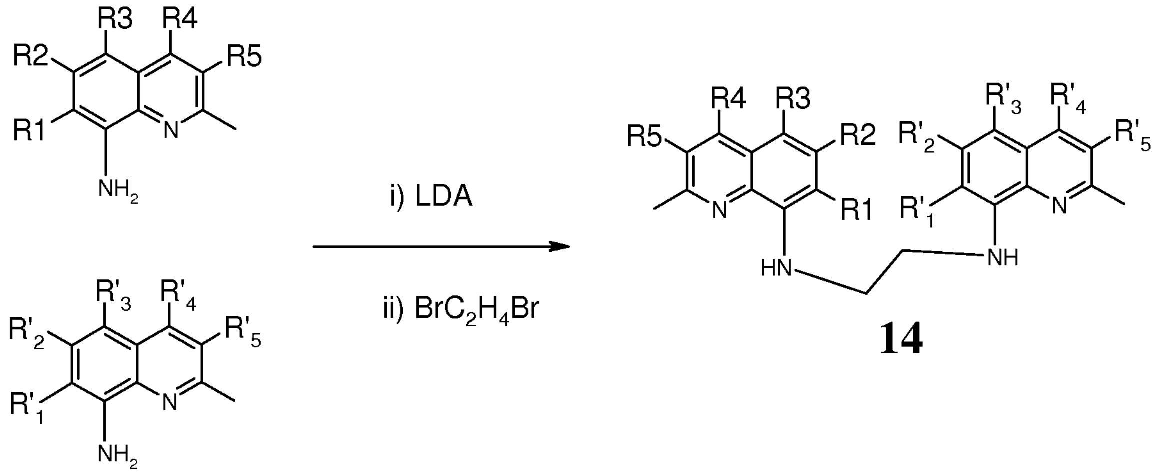


**14**

Schéma 7 : Synthèse de **14** et ses analogues.

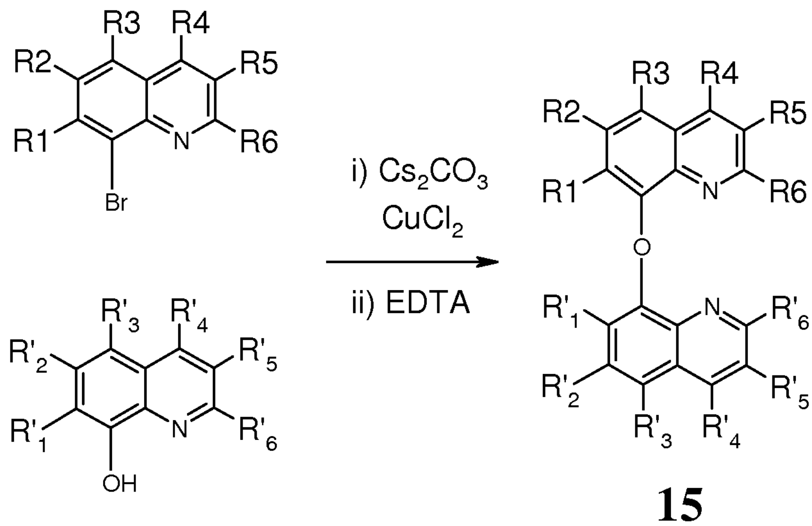


**15**

Schéma 8 : Synthèse de **15** et ses analogues.

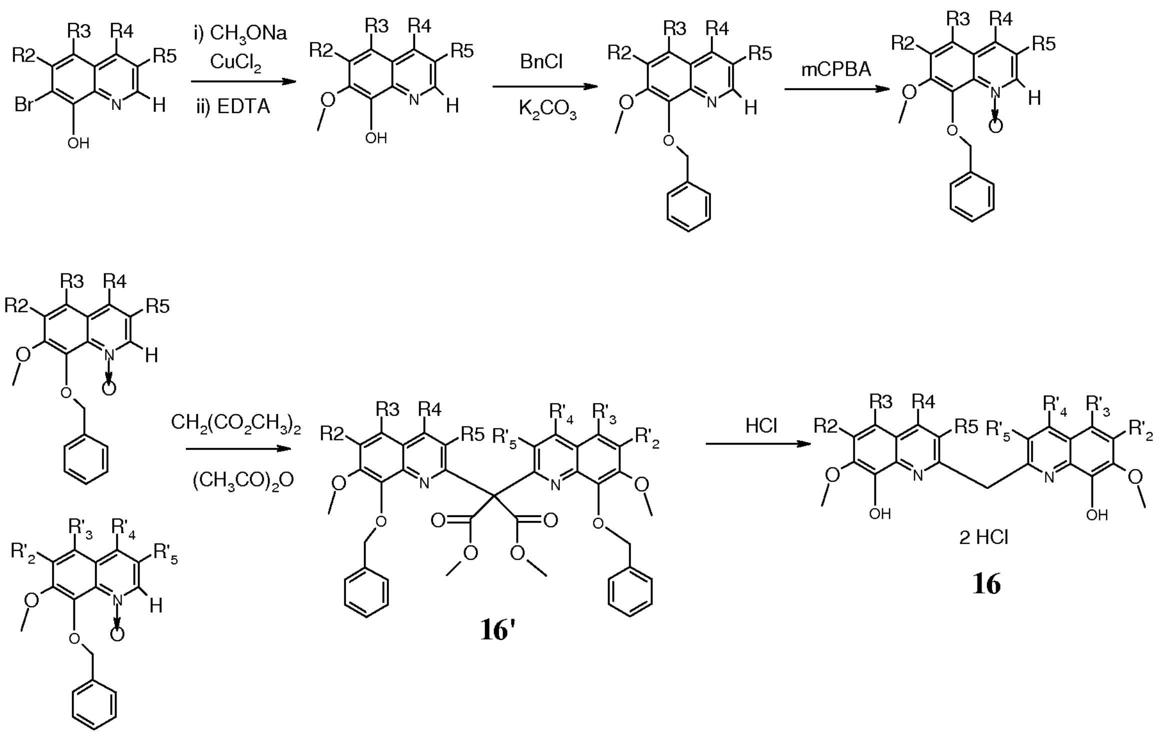


16'

16

Schéma 9 : Synthèse de **16'**, **16** et leurs analogues.

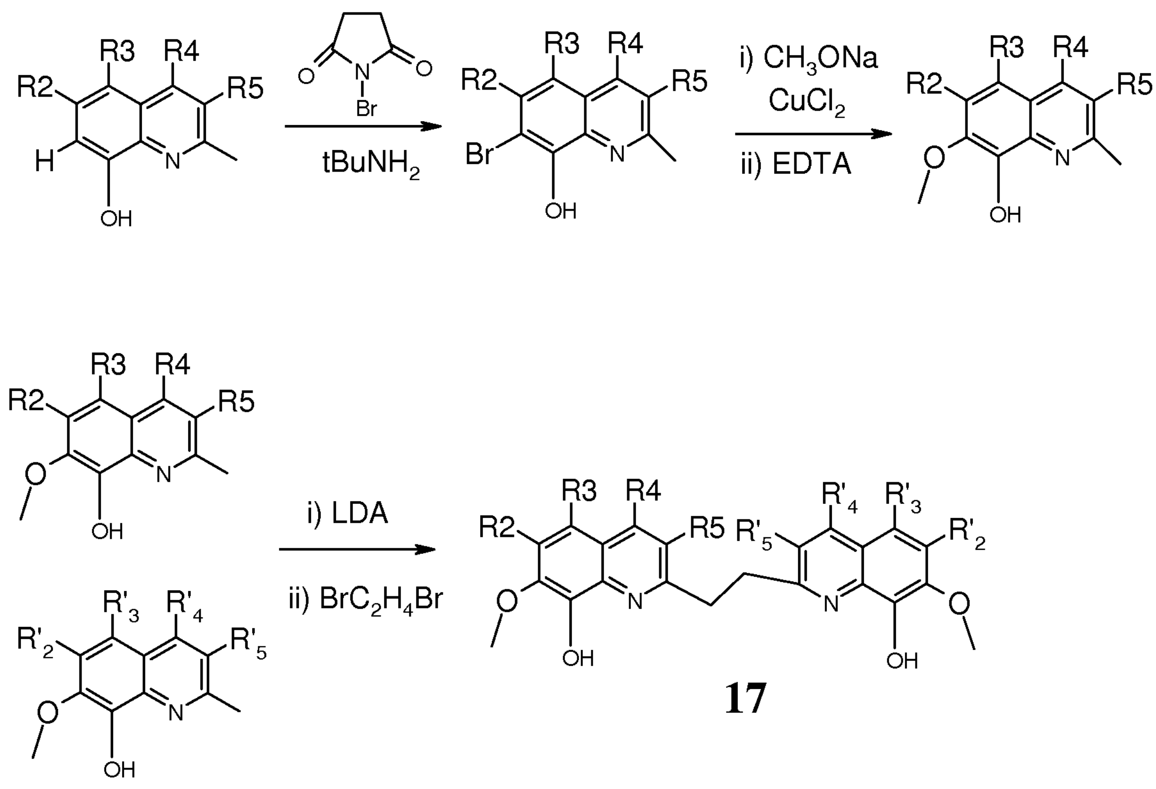


17

Schéma 10 : Synthèse de **17** et ses analogues.

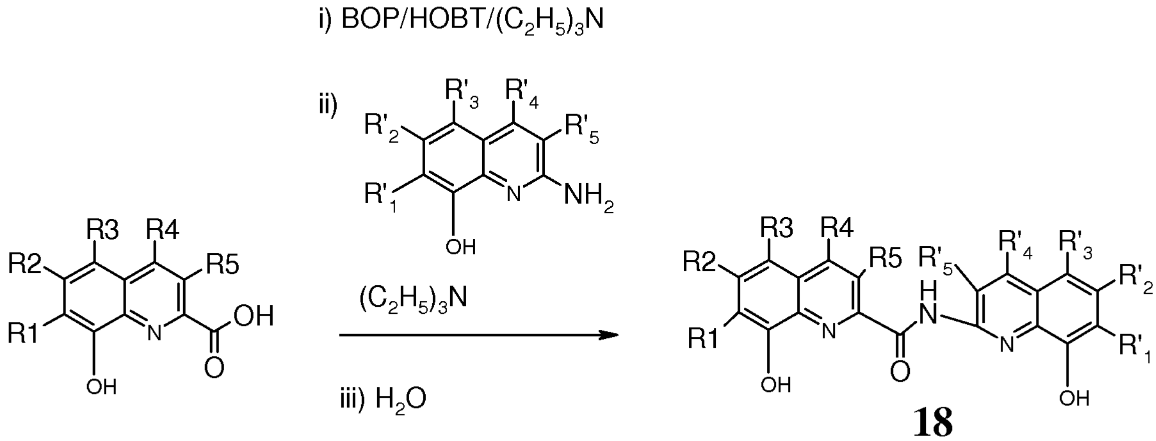


18

Schéma 11 : Synthèse de **18** et ses analogues.

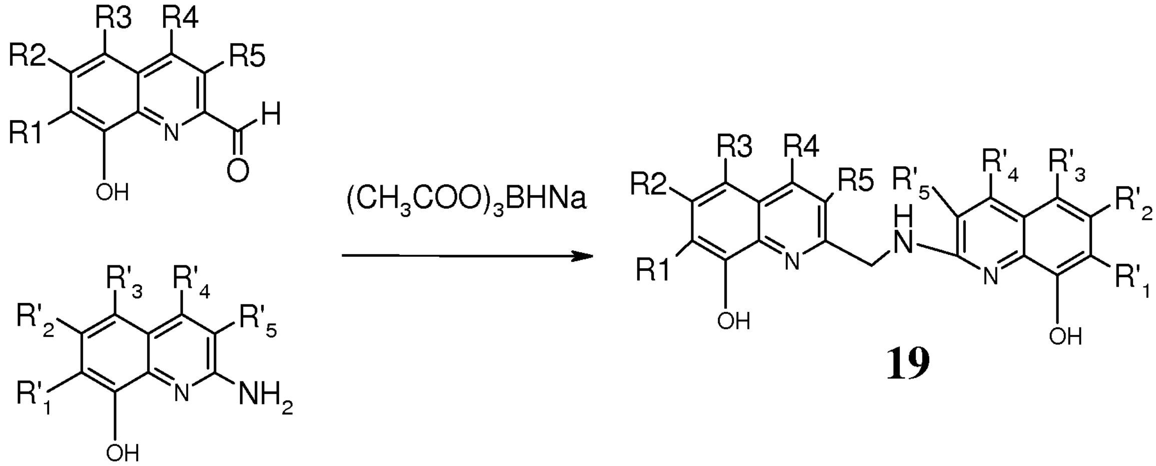


19

Schéma 12 : Synthèse de **19** et ses analogues.

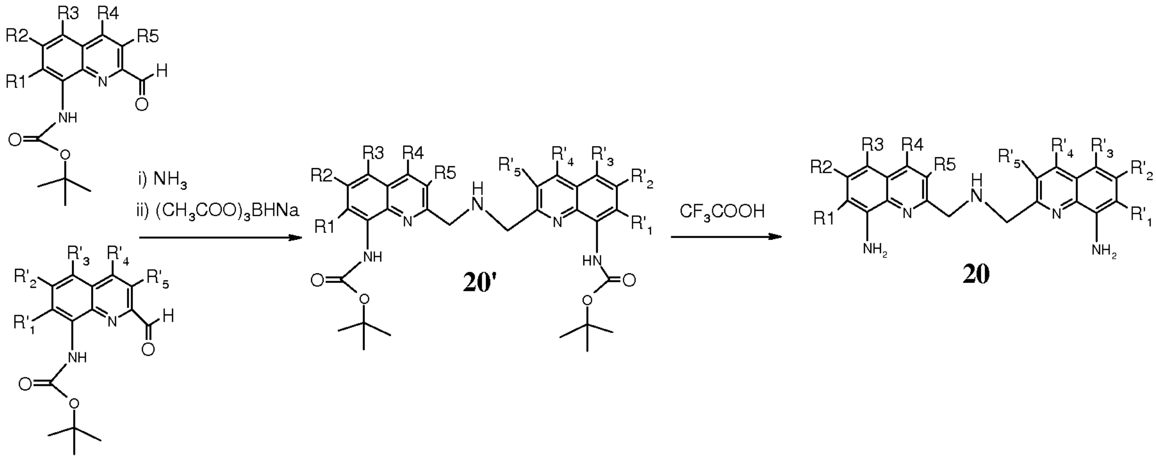


20'

20

Schéma 13 : Synthèse de **20'**, **20** et leurs analogues.

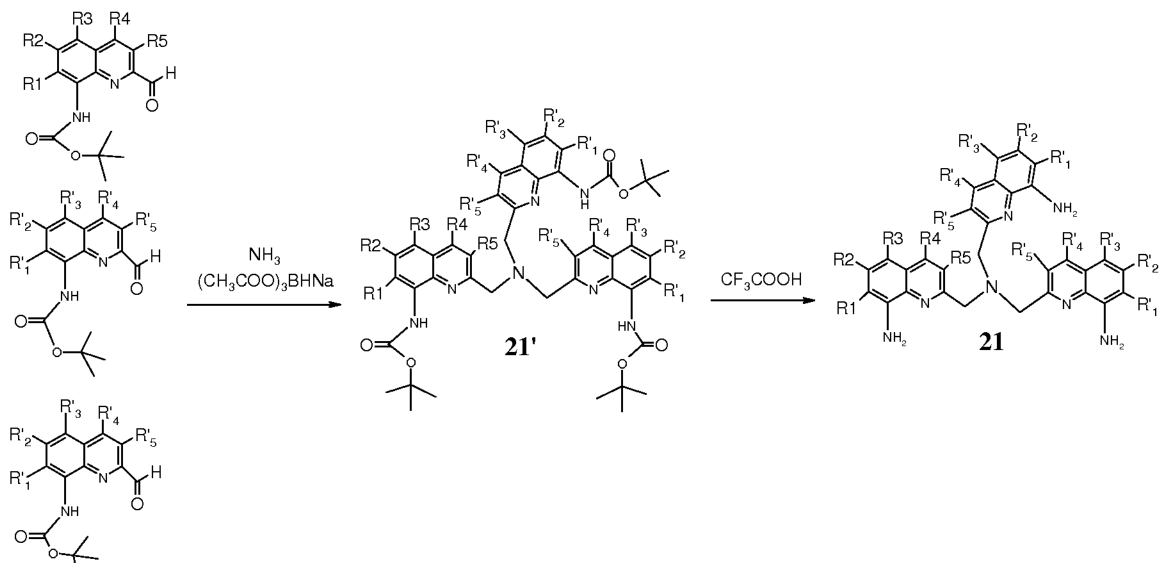


Schéma 14 : Synthèse de **21'**, **21** et leurs analogues.

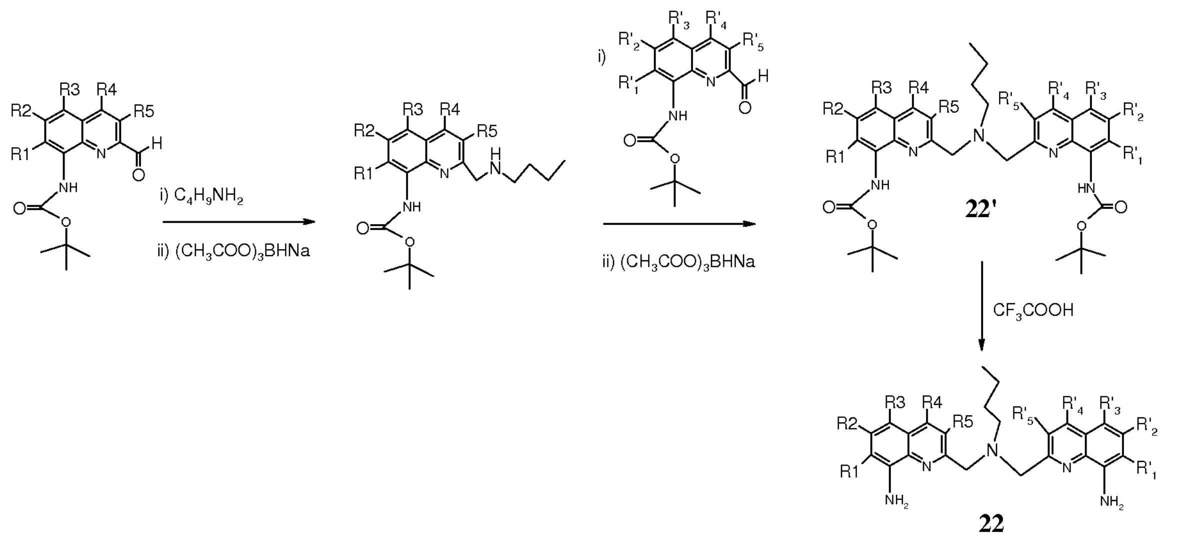


Schéma 15 : Synthèse de **22'**, **22** et leurs analogues.

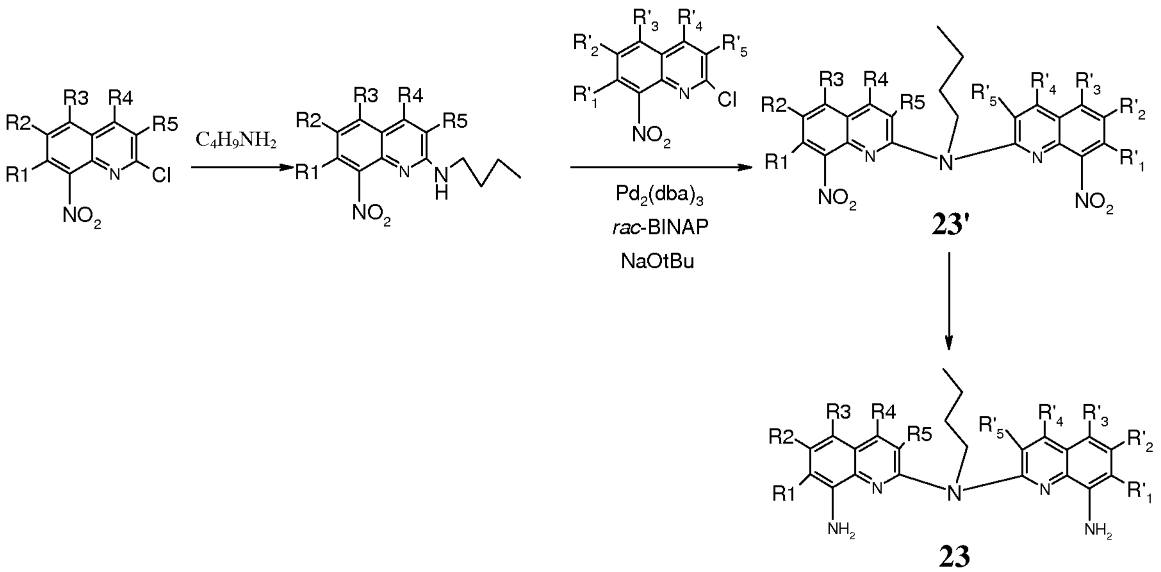


**23'**

**23**

Schéma 16 : Synthèse de **23'**, **23** et leurs analogues.

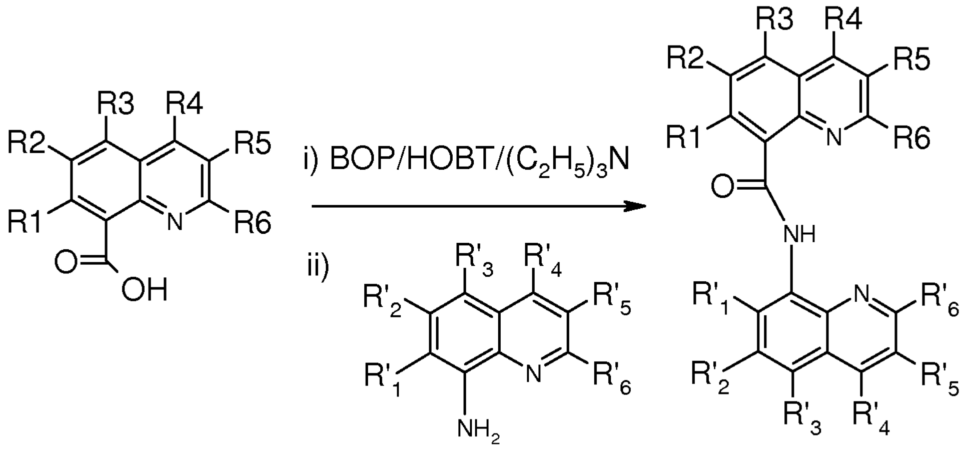


**24**

Schéma 17 : Synthèse de **24** et ses analogues.

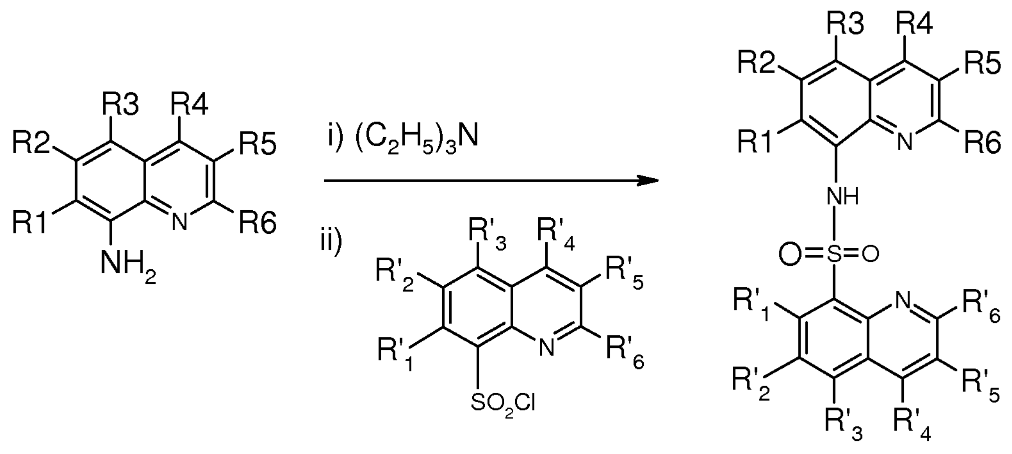


**25**

Schéma 18 : Synthèse de **25** et ses analogues.

Les intermédiaires réactionnels sont disponibles commercialement ou peuvent être préparés par l'homme du métier par application ou adaptation de méthodes connues en soi.

Le procédé selon l'invention peut également comprendre l'étape ultérieure d'isolation des produits de formule (I) obtenus.

Dans les réactions décrites ici, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Greene et P.G.M. Wuts dans Protective Groups in Organic Chemistry, John Wiley and Sons, 1991 ; J.F.W. McOmie in Protective Groups in Organic Chemistry, Plénum Press, 1973.

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention peuvent contenir des centres asymétriques. Ces centres asymétriques peuvent être indépendamment en configuration R ou S. Il apparaîtra à l'homme du métier que certains composés utiles selon l'invention peuvent également présenter une isomérie géométrique. On doit comprendre que la présente invention comprend des isomères géométriques individuels et des stéréoisomères et des mélanges de ceux-ci, incluant des mélanges racémiques, de composés de formule (I) ci-dessus. Ce type d'isomères peuvent être séparés de leurs mélanges, par l'application ou l'adaptation de procédés connus, par exemple des techniques de chromatographie ou des techniques de recristallisation, ou ils sont préparés séparément à partir des isomères appropriés de leurs intermédiaires.

Aux fins de ce texte, il est entendu que les formes tautomériques sont comprises dans la citation d'un groupe donné, par exemple thio/mercapto ou oxo/hydroxy.

Les sels d'additions acides sont formés avec les composés utiles selon l'invention dans lesquels une fonction de base tels qu'un groupe amino, alkylamino ou dialkylamino, ou encore pyridine, phénol est présente. Les sels d'addition acide pharmaceutiquement acceptables, c'est-à-dire non toxiques, sont préférés. Les sels sélectionnés sont choisis de façon optimale pour être compatibles avec les véhicules pharmaceutiques habituels et adaptés pour l'administration orale ou parentérale. Les sels d'addition acide des composés utiles selon cette invention peuvent être préparés par réaction de la base libre avec l'acide approprié, par l'application ou l'adaptation de procédés connus. Par exemple, les sels d'addition acide des composés utiles selon cette invention peuvent être préparés soit en dissolvant la base libre dans de l'eau ou dans une solution aqueuse alcoolisée ou des solvants adaptés contenant l'acide approprié et en isolant le sel en évaporant la solution, ou en faisant réagir la base libre et l'acide dans un solvant organique, auquel cas le sel se sépare directement ou peut être obtenu par concentration de la solution. Parmi les acides adaptés pour l'usage dans la préparation de ces sels on trouve acide chlorhydrique, acide bromhydrique, acide phosphorique, acide sulfurique, divers acides carboxyliques et sulfoniques organiques, tels que acide acétique, acide citrique, acide propionique, acide

succinique, acide benzoïque, acide tartrique, acide fumarique, acide mandélique, acide ascorbique, acide malique, acide méthanesulfonique, acide toluène-sulfonique, acides gras, adipate, alginate, ascorbate, aspartate, benzène-sulfonate, benzoate, propionate de cyclopentane, digluconate, dodécylsulfate, bisulfate, butyrate, lactate, laurate, sulfate de lauryle, malate, hydroiodide, 2-hydroxyéthanesulfonate, glycéro-phosphate, picrate, pivalate, palmoate, pectinate, persulfate, 3-phényl-propionate, thiocyanate, 2-naphtalène-sulfonate, undécanoate, nicotinate, hémisulfate, heptanoate, hexanoate, camphorate, camphresulfonate et autres.

Les sels d'addition acide des composés utiles selon cette invention peuvent être régénérés à partir des sels par l'application ou l'adaptation de procédés connus. Par exemple, les composés parents utiles selon l'invention peuvent être régénérés à partir de leurs sels d'addition acide par traitement avec un alkali, par exemple une solution de bicarbonate de sodium aqueuse ou une solution d'ammoniac aqueuse.

Les composés utiles selon cette invention peuvent être régénérés à partir de leurs sels d'addition de base par l'application ou l'adaptation de procédés connus. Par exemple, les composés parents utiles selon l'invention peuvent être régénérés à partir de leurs sels d'addition de base par le traitement avec un acide, par exemple un acide chlorhydrique.

Les sels d'addition de base peuvent être formés lorsque le composé utile selon l'invention contient un groupe carboxyle, ou encore pyridine ou phénol, ou un bioisostère suffisamment acide. Les bases qui peuvent être utilisées pour préparer les sels d'addition de base comprennent de préférence celle qui produisent, lorsqu'elles sont associées à un acide libre, des sels pharmaceutiquement acceptables, c'est-à-dire des sels dont les cations ne sont pas toxiques pour le patient dans les doses pharmaceutiques des sels, de sorte que les effets inhibiteurs bénéfiques inhérents à la base libre ne soient pas annulés par les effets secondaires imputables aux cations. Les sels pharmaceutiquement acceptables, comprenant ceux dérivés des sels de métal alcalino-terreux, dans la portée de l'invention comprennent ceux dérivés des bases suivantes : hydrure de sodium, hydroxyde de sodium, hydroxyde de potassium, hydroxyde de calcium, hydroxyde d'aluminium, hydroxyde de lithium, hydroxyde de magnésium, hydroxyde de zinc, ammoniac, éthylènediamine, *N*-méthyl-glucamine, lysine, arginine, ornithine, choline, *N,N'*-dibenzyl-éthylènediamine, chloroprocaïne, diéthanolamine, procaïne, *N*-benzylphénéthyl-amine, diéthylamine, pipérazine, tris(hydroxy-méthyl)aminométhane, hydroxyde de tétraméthylammonium et analogues.

Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation d'un mélange de solvant aqueux/organique, en utilisant des solvants organiques tels que dioxane, tétrahydrofurane ou méthanol.

Les produits de base ou les réactifs utilisés sont disponibles commercialement et/ou peuvent être préparés par l'application ou l'adaptation de procédés connus, par exemple des procédés tels que décrits dans les Exemples de Référence ou leurs équivalents chimiques évidents.

Selon la présente invention, les composés de formule (I) présentent une activité biologique de chélateur de métaux et/ou de dissolution d'agrégats amyloïdes.

La présente invention a également pour objet les compositions pharmaceutiques comprenant un composé selon l'invention avec un véhicule ou excipient pharmaceutiquement acceptable.

De préférence, ladite composition contient une quantité efficace du composé selon l'invention.

Selon un autre objet, la présente invention concerne également l'utilisation de composés de formule générale (I) pour la préparation de compositions pharmaceutiques destinées à chélater les ions métalliques, plus préférentiellement ceux de zinc, cuivre, fer, aluminium ou manganèse, plus préférentiellement les ions du zinc de degré d'oxydation II, du cuivre de degré d'oxydation I, II, du fer de degré d'oxydation II, III, IV ou V.

L'invention concerne également l'utilisation de composés de formule générale (I) pour la préparation de compositions pharmaceutiques destinées à dissoudre les agrégats amyloïdes.

Selon un autre objet, la présente invention concerne également l'utilisation de composés de formule générale (I) pour la préparation de compositions pharmaceutiques destinées à prévenir et/ou traiter, affectant le système nerveux central, telles que les maladies neuro-dégénératives. Plus particulièrement, les maladies neurodégénératives sont choisies parmi la maladie d'Alzheimer, la maladie de Parkinson, la maladie d'Huntington, la sclérose latérale amyotrophique ou la trisomie 21, les encéphalopathies spongiformes, telle que la maladie de Kreutzfeld-Jacob.

De préférence, ladite composition est administrée à un patient qui en a besoin.

Les compositions pharmaceutiques selon l'invention peuvent être présentées sous des formes destinées à l'administration par voie parentérale, orale, rectale, intraveineuse, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalino-terreux, le phosphate de magnésium, les amidons, les amidons modifiés, le lactose pour les formes solides.

Pour l'usage rectal, le beurre de cacao ou les stéarates de poly-éthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1000 mg) en fonction de l'indication thérapeutique et de la voie d'administration, ainsi que de l'âge et du poids du sujet.

Légendes des figures :

**Figure 1 :** Dérivés de quinoléine testés sur Aβ.
**Figure 2 :** Géométries *cis* et *trans* pour la complexation d'ions métalliques par les dérivés de 8-hydroxyquinoléine.
**Figure 3 :** Structure cristalline du [2,2'-(2,2-propanediyl)-bis[8-quinoléinolato]nickel(II); les atomes d'hydrogène ont été omis. Remarquer que le complexe est de configuration *cis* (Figure 2) et que les deux entités quinoléine du ligand chélatent le même atome métallique.
**Figure 4 :** Structure cristalline de bis{7-(méthyloxy)8-[phénylméthyl)oxy]-2-quinoléinyl}propanedioate, les atomes d'hydrogène ont été omis.
**Figure 5 :** Structure cristalline de 2-chloro-8-nitroquinoléine.
**Figure 6:** Spectres UV-visible obtenus lors de la titration Cu(II) par le ligand 3. Les spectres de titration sont obtenus avec 15 μM de ligand (L) dans le mélange tampon Tris ▪ HCl 20 mM ; NaCl 150 mM (pH = 7,4)/$CH_3OH$ (1/1, v/v) et différents rapports de $CuCl_2$. La direction des flèches indique les variations observées sur les spectres lorsque le rapport Cu(II)/L augmente.
**Figure 7:** Spectres UV-visible de **3 ;** Cu(II)-**3**; EDTA et (EDTA)Cu(II) dans le mélange tampon Tris·HCl 20 mM ; NaCl 150 mM (pH = 7,4)/$CH_3OH$ (1/1, v/v). La concentration des ligands et des complexes métalliques est de 15 μM. Remarquer qu'une absorbance à λ = 379 nm est seulement observée pour le complexe Cu(II)-**3**.
**Figure 8:** Réaction de compétition pour la complexation de $CuCl_2$ par le ligand **3** ($L_s$) et EDTA ($L_c$).
Les spectres sont obtenus avec 15 μM de **3** et de $CuCl_2$ et différents rapports de EDTA dans le mélange 20 mM tampon Tris·HCl/150 mM NaCl (pH = 7,4)/$CH_3OH$ (1/1, v/v). La direction des flèches indique les variations d'absorbance observées quand le rapport $L_c/L_s$ augmente.
**Figure 9:** Variation du pourcentage de peptide $A\beta_{1-42}$ soluble en fonction de la stoechiométrie Aβ/Cu(II). Le peptide $A\beta_{1-42}$ (5 μM) est incubé durant deux heures à 37 °C dans du tampon Tris·HCl 20 mM (pH = 7,4); NaCl 150 mM en présence de différentes stoechiométries de Cu(II) puis le mélange réactionnel est centrifugé. Le pourcentage de peptide dans le surnageant ($A\beta_{1-42}$ soluble) et dans le précipité ($A\beta_{1-42}$ agrégé) sont alors quantifiés à l'aide d'un kit Micro BCA Protein Assays. Valeur 1 et Valeur 2 sont issues de deux expériences indépendantes, quand le nombre de mesures est ≥ 3, la moyenne et l'écart type des valeurs obtenues sont présentés.
**Figure 10:** Variation du pourcentage de peptide $AB_{1-42}$ soluble en présence de différentes stoechiométries de $CuCl_2$ et de ligands. Le peptide $A\beta_{1-42}$ (5 μM) est incubé durant une heure à 37 °C dans du tampon Tris·HCl 20 mM (pH = 7,4); NaCl 150 mM en présence de $CuCl_2$ 12,5 ou 20 μM puis le ligand [12,5 μM (sauf pour les monomères de quinoléine comme 8-hydroxyquinoléine, Clioquinol, 8-hydroxyquinaldine et 8-aminoquinoléine où cette valeur est de 25 μM) ou 200 μM] est ajouté. Après une heure supplémentaire d'incubation, le mélange réactionnel est centrifugé. Le pourcentage de peptide dans le surnageant ($AP_{1-42}$ soluble) et dans le précipité ($AP_{1-42}$ agrégé) sont alors quantifiés à l'aide d'un kit Micro BCA Protein Assays. Pour chaque composé, les valeurs de gauche (en gris foncé) et de droite (en gris clair) représentent les résultats obtenus pour respectivement les rapports 200 μM de ligand/20 μM $CuCl_2$ et 12,5 μM (dans le cas des dimères de quinoléine ou 25 μM dans le cas des monomères) de ligand/12,5 μM $CuCl_2$. Sont aussi indiqués les pourcentages de peptide $A\beta_{1-42}$ soluble en présence des différentes concentrations de $CuCl_2$ en l'absence de ligand.
**Figure 11:** Variation de la production de $H_2O_2$ par les complexes de cuivre du peptide $A\beta_{1-42}$ en fonction de la stoechiométrie Cu(II)/Aβ. Les expériences sont réalisées durant 5 minutes dans du tampon phosphate de sodium (pH = 7,4) en présence de 0,4 μM $CuCl_2$, 10 μM ascorbate et d'air avec ou sans 0,4 μM de **3** ou de **7.** Le $H_2O_2$ libéré est quantifié à l'aide d'un kit Amplex-Red $H_2O_2$/HRP Assay.

Les exemples suivants illustrent l'invention, sans toutefois la limiter. Les produits de départ utilisés sont des produits connus ou préparés selon des modes opératoires connus.

Les abréviations suivantes ont été utilisées ci-avant ou ci-après :

Aβ: peptide β-amyloïde; BSA: Albumine de Sérum Bovin; biPy: 2,2'-bipyridine; BnCl : chlorure de benzyle; BOP: (benzotriazole-1-yloxy)tris(diméthylamino)phosphonium hexafluorophosphate; CTDA: acide *trans*-1,2-diaminocyclohexane-*N,N,N,N'*-tétraacétique; d: doublet; dd : doublet dédoublé ; DIC : détection par ionisation chimique ; Dien:

diéthylènetriamine; DMF : diméthylformamide ; DMSO : diméthylsulfoxide ; DTPA: acide diéthylène-triaminepentaacétique; EDA: éthylènediamine; EDDA: acide éthylènediamine-*N,N'*-diacétique; EDTA: acide éthylènediaminetétraacétique; EGTA: acide éthylènebis(oxyéthylènenitrilo)-tétraacétique; eq: équivalent; Eq. : équation ; HOBT: 1-hydroxybenzotriazole monohydrate; HRP: péroxidase de raifort (Horse Radish Peroxidase); HIDA: acide *N*-(2-hydroéthyl)iminodiacétique; LDA: diisopropylamine de lithium; m : massif ; mCPBA: acide *m*-chloroperbenzoïque; NBS: *N*-bromosuccinimide; NTA: acide nitrilotriacétique; $Pd_2(dba)_2$ : tris(dibenzylidène-acétone)-dipalladium(0); PM : poids moléculaire ; *rac*-BINAP: *rac*-2,2'-bis(diphénylphosphino)-1,1',binaphthyl; s : singulet ; SM : spectrométrie de masse ; t : triplet ; Tetren: tétraéthylènepentamine; THF : tétrahydrofurane ; Trien: triéthylènetétramine ; t/min : tours par minute.

EXEMPLES

Description des exemples :

**Synthèse :**

***Remarques générales concernant la synthèse organique :***

8-méthyloxyquinaldine a été synthétisé suivant une méthode décrite dans la littérature. (C. Kitamura et al., J. Chem. Soc., Perkin Trans. 1 2000, 781-785). *N*-butyl-2,2'-imino-bis(8-nitroquinoléine) a été synthétisée selon les références: H. Jiang et al. Tetrahedron 2003, 59, 8365-8374; G. Xue et al., Tetrahedron 2001, 57, 7623-7628 et P. Belser et al. Tetrahedron 1996, 52, 2937-2944. 7-bromo-8-hydroxyquinoléine (préparée selon la méthode de Pearson: D.E. Pearson et al. J. Org. Chem. 1967, 32, 2358-2360; G. E. Collis et al. Acta Cryst. 2003, C59, o443-o444). 1,1-diméthyl(2-formyl-8-quinoléinyl)carbamate a été synthétisé selon G. Xue et al., Tetrahedron 2001, 57, 7623-7628. 2-Chloro-8-nitroquinoléine a été synthétisé selon M. C. Kimber et al., Aust. J. Chem. 2003, 56, 39-44.

L'acétonitrile a été séché sur tamis moléculaire 4 Å. Le tétrahydrofurane (THF) a été distillé sur de la benzophénone en présence de sodium. $CuCl_2$ sec a été obtenu par chauffage sous vide à 50 °C. Le dicflorométhane a été séché sur allumine basique. Les autres réactifs et solvants utilisés proviennent de fournisseurs standard en produits chimiques et ont été utilisés sans purification ultérieure.

Les spectres RMN ont été enregistrés sur des appareils Bruker 200, 250 ou 500 MHz. Le spectromètre de masse en ionisation électrospray (IES-SM) était un appareil Perkin-Elmer SCIEX API 365, les échantillons ont été introduits dans la source électrospray avec une pompe à seringue Havard Apparatus. Les spectres UV-visible ont été enregistrés sur un spectrophotomètre à barrette de diodes Hewlett Packard 8452A ou un spectrophotomètre Perkin-Elmer Lamda 35.

Les chromatographies couche mince sont réalisées sur plaque de silice.

**2-Chloro-8-nitroquinoléine :**

La synthèse a été réalisée selon le protocole de: M. C. Kimber et al., *Aust. J. Chem* **2003**, *56*, 39-44. Des données de RMN plus précises que celles préalablement publiées sont ajoutées ci-après. RMN-$^1$H (250 MHz, $CDCl_3$) d, ppm: 8,21 (d, $^3J$ (H, H) = 8,5 Hz, 1 H); 8,10 (dd, $^3J$ (H, H) = 7,5 Hz, $^4J$ (H, H) = 1,5 Hz, 1 H); 8,05 (dd, $^3J$ (H, H) = 8,0 Hz, $^4$J (H, H) = 1,5 Hz, 1 H); 7,65 (m, 1 H); 7,55 (d, $^3J$ (H, H) = 8,5 Hz, 1 H). RMN-$^{13}$C (63 MHz, $CDCl_3$) $\delta$, ppm: 153,5 (Cq); 147,1 (Cq); 138,9 (Cq); 138,8 (CH); 131,9 (CH); 127,6 (Cq); 125,8 (CH); 125,0 (CH); 124,5 (CH). SM (DIC, $NH_3$): m/z = 209 ($MH^+$), 226 ($MNH_4^+$), 243 ($MN_2H_7^+$). Analyse (%) pour $C_9H_5N_2O_2Cl$: calculé C 51,82; H 2,42; N 13,43; trouvé C 51,76; H 2,37; N 13,24. La structure de la molécule a été confirmée par analyse par diffraction de rayons X sur des monocristaux obtenus par cristallisation du produit dans du chloroforme deutéré. La structure est présentée Figure 5. Les paramètres de l'analyse cristalline sont les suivants: système cristallin orthorhombique ; P c a 21; a = 18,090 (4) Å, b = 3,7781 (7) Å, c = 12,581 (2) Å, $\alpha$ = 90°,$\beta$ = 90°,$\gamma$= 90°.

***N*-Butyl-8-nitro-2-quinoléinamine :**

2-Chloro-8-nitroquinoléine (0,20 g; 0,96 mmol) est mis en suspension dans 9,5 mL de 1-butylamine et le milieu est chauffé au reflux (78 °C) pendant 15 heures. La solution jaune obtenue est ensuite concentrée sous vide. Le brut réactionnel est repris dans un volume minimum de $CH_3OH$ et versé sur 3 mL de diéthyéther. Après centrifugation, des cristaux de chlorure de butylammonium sont éliminés et le surnageant est concentré sous pression réduite pour donner N-butyl-8-nitro-2-quinoléinamine sous la forme d'une huile jaune (0,21 g; 0,86 mmol, rendement = 90%). RMN-$^1$H (250 MHz, $CDCl_3$) $\delta$, ppm: 7,83 (dd, $^3J$ (H, H) = 7,5 Hz, $^4J$ (H, H) = 1,5 Hz, 1 H); 7,77 (d, $^3J$ (H, H) = 9,0 Hz, 1 H); 7,69 (dd, $^3J$ (H, H) = 8,0 Hz, $^4J$ (H, H) = 1,5 Hz, 1 H); 7,14 (m, 1 H), 6,67 (d, $^3J$ (H, H) = 9,0 Hz, 1 H); 5,07 (s large, 1 H); 3,47 (m,

2 H); 1,60 (m, 2 H); 1,39 (m, 2 H); 0,93 (t, $^3J$ (H, H) = 7,5 Hz, 3 H). RMN-$^{13}$C (63 MHz, $CDCl_3$) δ, ppm: 157,8 (Cq); 145,6 (Cq); 140,2 (Cq); 136,7 (CH); 131,5 (CH); 124,7 (CH); 124,1 (CH); 119,7 (CH); 113,3 (Cq); 41,3 ($CH_2$); 31,5 ($CH_2$); 20,2 ($CH_2$); 13,8 ($CH_3$). SM (DIC, $NH_3$): m/z = 246 ($MH^+$). Analyse (%) pour $C_{13}H_{15}N_3O_2$: calculé C 63,66; H 6,16; N 17,13; trouvé C 63,33; H 6,21; N 16,64.

***N*-Butyl-2,2'-imino-bis(8-nitroquinoléine) (composé 23'):**

A une suspension violette de N-butyl-8-nitro-2-quinoléinamine (0,17 g; 0,69 mmol), de tris(dibenzylidène-acétone)-dipalladium(0) (13 mg; 0,014 mmol) et de *rac*-2,2'-bis(diphénylphosphino)-1,1'-binaphthyl (17 mg; 0,027 mmol) dans 5 mL de toluène sous argon est ajouté 2-chloro-8-nitroquinoléine (122 mg; 0,59 mmol) et NaOtBu (77,5 mg; 0,81 mmol). Le milieu est chauffé au reflux pendant 3 heures, puis du 2-chloro-8-nitroquinoléine (22 mg; 0,11 mmol) est à nouveau ajouté. Le chauffage est poursuivi pendant 2 heures 30 min puis 10 mL d'une solution saturée de chlorure d'ammonium sont ajoutés. Le produit est extrait avec 3 × 30 mL de $CH_2Cl_2$ et le solvant est évaporé sous pression réduite. Le produit est purifié par chromatographie sur gel de silice, élué avec $CH_2Cl_2$/hexane (80/20, v/v) pour donner N-butyl-2,2'-imino-bis(8-nitroquinoléine) sous la forme d'une poudre jaune (0,11 g; 0,26 mmol, rendement = 38 %). RMN-$^1$H (250 MHz, $CDCl_3$) δ, ppm: 8,14 (d, $^3J$ (H, H) = 9,0 Hz, 2 H); 7,99 (dd, $^3J$ (H, H) = 7,5 Hz, $^4J$ (H, H) = 1,5 Hz, 2 H); 7,93 (dd, $^3J$ (H, H) = 8,0 Hz, $^4J$ (H, H) = 1,5 Hz, 2 H); 7,73 (d, $^3J$ (H, H) = 9,0 Hz ,2 H); 7,43 (m, 2 H); 4,45 (t, $^3J$ (H, H) = 7,5 Hz, 2 H); 1,83 (m, 2 H); 1,47 (m, 2 H); 0,98 (t, $^3J$ (H, H) = 7,5 Hz, 3 H). RMN-$^{13}$C (63 MHz, $CDCl_3$) δ, ppm: 156,2 (Cq); 146,4 (Cq); 138,7 (Cq); 137,0 (CH); 131,5 (CH); 126,2 (Cq); 124,2 (CH); 122,8 (CH); 117,3 (CH); 48,9 ($CH_2$); 30,4 ($CH_2$); 20,3 ($CH_2$); 13,9 ($CH_3$). SM (DIC, $NH_3$): m/z = 418 ($MH^+$). Analyse (%) pour $C_{22}H_{19}N_5O_4$: calculé C 63,30; H 4,59; N 16,78; trouvé C 63,18; H 4,49; N 16,39. UV/vis [$CH_3OH$/Tris ▪ HCl 20 mM pH = 7,4; NaCl 150 mM (1/1, v/v)]: λ nm (ε $M^{-1}$ $cm^{-1}$) = 210 (91 400), 226 (53 500, épaulement), 270 (42 100), 294 (26 700, épaulement), 383 (28 400).

***N*-Butyl-2,2'-imino-bis(8-quinoléinamine) (composé 23)**

A une solution de N-butyl-2,2'-imino-bis(8-nitroquinoléine) (230 mg; 0,55 mmol) dans 35 mL d'acétate d'éthyle est ajouté du palladium sur charbon à 10% en masse (50 mg). Le mélange est placé sous atmosphère de dihydrogène (1 bar) et agité pendant 4 heures à température ambiante. Après élimination du palladium par filtration sur célite, la phase organique est concentrée sous pression réduite. Le brut réactionnel est repris dans le minimum de $CH_2Cl_2$ et précipité par 4 volumes d'hexane. Après filtration, le surnageant est concentré sous pression réduite et le produit est séché sous vide pour donner **23** sous la forme d'une poudre brune (197 mg; 0,55 mmol, rendement quantitatif). RMN-$^1$H (250 MHz, $CDCl_3$) δ, ppm: 7,90 (d, $^3J$ (H, H) = 9,0 Hz, 2 H); 7,32 (d, $^3J$ (H, H) = 9,0 Hz, 2 H); 7,20 (m, 2 H); 7,10 (dd, $^3J$ (H, H) = 8,0 Hz, $^4J$ (H, H) = 1,0 Hz, 2 H); 6,93 (dd, $^3J$ (H, H) = 7,5 Hz, $^4J$ (H, H) = 1,0 Hz, 2 H); 4,60 (s, 4H); 4,48 (t, $^3J$ (H, H) = 7,5 Hz, 2 H); 1,88 (m, 2 H); 1,47 (m, 2 H); 0,97 (t, $^3J$ (H, H) = 7,5 Hz, 3 H). RMN-$^{13}$C (63 MHz, $CDCl_3$) δ, ppm: 154,1 (Cq); 142,1 (Cq); 137,2 (CH); 137,0 (Cq); 125,3 (Cq); 125,0 (CH); 116,3 (CH); 116,1 (CH); 111,3 (CH); 48,9 ($CH_2$); 30,6 ($CH_2$); 20,7 ($CH_2$); 14,1 ($CH_3$). SM (DIC, $NH_3$): m/z = 358 ($MH^+$). UV/vis [$CH_3OH$/Tris ▪ HCl 20 mM pH = 7,4; NaCl 150 mM (1/1, v/v)]: λ nm (ε $M^{-1}$ $cm^{-1}$) = 211 (53 400, épaulement), 302 (40 300), 367 (20 000), 382 (14 600, épaulement).

**RESULTATS:**

**Capacité des composés à chélater les métaux:** (ces résultats concernent non seulement le composé 23 de l'invention, mais aussi des composés similaires qui ne répondent pas à la formule (I))

***Détermination de la stoechiométrie métal/ligand :***

Les spectres d'absorption UV-visible et les titrations spectrophotométriques UV-visible ont été réalisées en présence de tampon Tris·HCl 20 mM pH = 7,4 ; NaCl 150 mM puisque c'est le solvant utilisé durant les essais de resolubilisation de $A\beta_{1-42}$. Un solvant organique ($CH_3OH$, dioxane ou DMSO) a été ajouté afin d'avoir une bonne solubilité des ligands et des complexes métalliques aux concentrations utilisées dans ces expériences. Les proportions exactes du mélange solvant organique/tampon sont précisées au niveau de chaque caractérisation UV-visible des différents ligands ou complexes métalliques.

A une solution 15 μM de ligand sont ajoutés des aliquots de solutions concentrées de $CuCl_2$ ou de $ZnCl_2$ (M) afin de n'induire que des variations négligeables de volume. Après chaque addition, des changements des spectres d'absorption sont immédiatement observés et sont stables entre deux additions ce qui est en accord avec un processus rapide de complexation. Les valeurs exactes d'absorption concernant chaque ligand et ses complexes de cuivre(II) ou de zinc(II) sont détaillées par la suite dans la partie expérimentale (dans la partie synthèse pour les ligands et ci-dessous pour les complexes métalliques).

La stoechiométrie des différents ligands (L) pour Cu(II) ou Zn(II) a ainsi été déterminée spectrophotométriquement par titration de l'ion métallique. Un exemple typique, obtenu dans le cas de la titration de $CuCl_2$ par le ligand **3** est présenté sur la Figure 6. Dans cet exemple, pour un rapport M/L augmentant de 0 à 1, la transition $\pi \rightarrow \pi^*$, centrée à 251 nm pour le ligand libre, est déplacée vers une énergie plus basse avec une apparition concomitante d'une bande d'absorption dans la région visible du spectre ($\lambda_{max}$ à 383 nm) qui est probablement due à une transition MLCT. Pour des rapports M/L supérieurs, aucun changement supplémentaire dans les spectres d'absorption UV-visible n'est observé. Ces résultats sont en accord avec une capacité du ligand **3** à former un seul type de complexe de Cu(II) avec une stoechiométrie M/L (1/1).

Des résultats analogues on été obtenus avec d'autres ligands en présence de Cu(II) ou de Zn(II), ils sont repris dans le Tableau 3.

***Préparation des complexes métalliques pour la caractérisation en spectrométrie de masse :***

Les ligands, dissouts dans du méthanol ou du dioxane, ont été métallés en présence d'un équivalent d'ion métallique durant une heure à température ambiante puis le solvant est évaporé. Du $Cu(AcO)_2$ ou du $Zn(AcO)_2$ ont été employés pour les ligands **1** à **11** et du $CuCl_2$ ou du $ZnCl_2$ pour les autres ligands. Des analyses de contrôle, réalisées après redissolution des complexes dans le mélange tampon/solvant organique adéquat, ont montré que les spectres UV-visible des différents complexes ainsi obtenus sont les mêmes que ceux obtenus durant les expériences de titration pour une stoechiométrie identique de ligand et d'ion métallique.

**Cu(II)-1:** SM (DIC, $NH_3$) m/z: 364 (LCu(II) - 1 H), 381 (LCu(II)$NH_4$ - 2 H). UV/vis [dioxane/Tris ▪ HCl 20 mM pH = 7,4 ; NaCl 150 mM (1/1, v/v)]: $\lambda$ nm ($\varepsilon$ $M^{-1}$ $cm^{-1}$) = 256 (41 900), 272 (32 200, épaulement), 304 (8 800), 338 (5 100), 376 (3 100).

**Cu(II)-2:** SM (DIC, $NH_3$) m/z: 434 (LCu(II) - 1 H), 451 (LCu(II)$NH_4$ - 2 H). Analyse (%) pour $C_{19}H_{10}N_2O_2Cl_2Cu$: calculé C 52,73; H 2,33; N 6,47; trouvé C 52,52; H 1,64; N 6,48. UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 256 (38 300), 278 (35 900), 310 (10 300), 342 (7 300), 398 (4 000).

**Cu(II)-3 :** SM (DIC, $NH_3$) m/z: 392 (LCu(II) - 1 H), 409 (LCu(II)$NH_4$ - 2 H). Analyse (%) pour $C_{21}H_{16}N_2O_2Cu \cdot 0,3\ C_2H_4O_2$: calculé C 63,33; H 4,16; N 6,84; trouvé C 63,30; H 3,50; N 6,82. UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 202 (70 800), 254 (58 900), 268 (42 400, épaulement), 383 (4 300).

**Cu(II)-4 :** SM (DIC, $NH_3$) m/z: 462 (LCu(II) - 1 H), 479 (LCu(II)$NH_4$ - 2 H). Analyse (%) pour $C_{21}H_{14}N_2O_2Cl_2Cu$: calculé C 54,74; H 3,06; N 6,08; trouvé C 54,50; H 2,84; N 5,92. UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 261 (59 500), 274 (46 900, épaulement), 347 (4 700), 409 (5 800).

**Cu(II)-5:** SM (DIC, $NH_3$) m/z: 400 (LCu(II) - 1 H), 417 (LCu(II)$NH_4$ - 2 H). UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 205 (64 000), 255 (47 600), 276 (34 400), 415 (2 900).

**Cu(II)-6 :** SM (DIC, $NH_3$) m/z: 378 (LCu(II) - 1 H), 395 (LCu(II)$NH_4$ - 2 H). UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 245 (41 100), 306 (27 900), 348 (12 100, épaulement), 485 (2 500).

**Cu(II)-7 :** SM (DIC, $NH_3$) m/z: 378 (LCu(II) - 1 H)395 (LCu(II)$NH_4$ - 2 H). Analyse (%) pour $C_{20}H_{14}N_2O_2Cu \cdot 0,3\ H_2O$: calculé C 62,67; H 3,84; N 7,31; trouvé C 62,68; H 2,91; N 7,21. UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 202 (57 700), 259 (68 000), 268 (46 200, épaulement), 392 (4 900).

**Cu(II)-8 :** SM (DIC, $NH_3$) m/z: 448 (LCu(II) - 1 H), 465 (LCu(II)$NH_4$ - 2 H). Analyse (%) pour $C_{20}H_{12}N_2O_2Cl_2Cu \cdot 0,3\ H_2O$: calculé C 53,12; H 2,81; N 6,20; trouvé C 53,09; H 2,37; N 6,08. UV/vis [dioxane/Tris·HCl 20 mM pH = 7,4 ; NaCl 150 mM (1/1, v/v)]: $\lambda$ nm ($\varepsilon$ $M^{-1}$ $cm^{-1}$) = 264 (64 700), 276 (46 700), 413 (5 300).

**Cu(II)-9 :** SM (DIC, $NH_3$) m/z: 700 (LCu(II) - 1 H), 717 (LCu(II)$NH_4$ - 2 H). Anal. pour $C_{20}H_{10}N_2O_2Cl_2I_2Cu \cdot 2(C_2H_4O_2)$: calculé C 35,30; H 1,97; N 3,43; trouvé C 35,46; H 1,20; N 3,83. UV/vis [DMSO/Tris·HCl 20 mM pH = 7,4 ; NaCl 150 mM (8/2, v/v)]: $\lambda$ nm ($\varepsilon$ $M^{-1}$ $cm^{-1}$) = (56 000), 340 (55 000), 392 (6 200), 420 (5 000).

**Cu(II)-10:** SM (DIC, $NH_3$) m/z: 406 (LCu(II) - 1 H), 423 (LCu(II)$NH_4$ - 2 H). UV/vis [$CH_3OH$/Tris·HCl 20 mM pH = 7,4 ; NaCl 150 mM (1/1, v/v)]: $\lambda$ nm ($\varepsilon$ $M^{-1}$ $cm^{-1}$) = 204 (56 000), 260 (71 800), 374 (4 800).

**Cu(II)-12-Cl :** SM (DIC, $NH_3$) m/z: 369 (LCu(II)Cl). UV/vis [DMSO/Tris·HCl 20 mM pH = 7,4 ; NaCl 150 mM (8/2, v/v)]: $\lambda$ nm ($\varepsilon$ $M^{-1}$ $cm^{-1}$) = 287 (40 700), 368 (3 300), 486 (14 100).

**Cu(II)-13:** SM (électrospray, > 0) m/z: 390 (LCu(II) - 1 H). UV/vis [$CH_3OH$/Tris·HCl 20 mM pH = 7,4; NaCl 150 mM (1/1, v/v)]: $\lambda$nm ($\varepsilon$ $M^{-1}$ $cm^{-1}$) = 230 (55 700); 303 (11 800), 315 (10 400), 360 (1 100, épaulement).

**Cu(II)-14 :** SM (électrospray, > 0) m/z: 405 [LCu(II)].

**Cu(II)-16:** SM (électrospray, > 0) m/z: 423 (LCu(II) - 2 H). UV/vis [$CH_3oH$/Tris·HCl 20 mM pH = 7,4; NaCl 150 mM (1/1, v/v)]: $\lambda$ nm ($\varepsilon$ $M^{-1}$ $cm^{-1}$) = 254 (34 400), 278 (35 900), 316 (9 700, épaulement), 380 (3 100).

**Cu(II)-17 :** SM (électrospray, > 0) m/z: 438 (LCu(II) - 1 H). UV/vis [$CH_3OH$/Tris·HCl 20 mM pH = 7,4; NaCl 150 mM (1/1, v/v)]: $\lambda$ nm ($\varepsilon$ $M^{-1}$ $cm^{-1}$) = 212 (50 300), 263 (65 300), 275 (47 300, épaulement), 319 (4 500), 421 (5 900).

**Cu(II)-23:** SM (électrospray, > 0) m/z: 419 (LCu(II) - 1 H). UV/vis [$CH_3OH$/Tris·HCl 20 mM pH = 7,4; NaCl 150 mM (1/1, v/v)]: $\lambda$ nm ($\varepsilon$ $M^{-1}$ $cm^{-1}$) = 211 (65 400); 235 (33 800, épaulement), 275 (37 000), 302 (5 900, épaulement), 329 (17 900), 352 (19 000), 366 (20 900).

**Cu(II)-24 :** SM (électrospray, > 0) m/z: 361 (LCu(II) - 1 H).

**Zn(II)-1 :** SM (DIC, $NH_3$) m/z: 365 (LZn(II) - 1 H), 382 (LZn(II)$NH_4$ - 2 H).

**Zn(II)-2 :** SM (DIC, $NH_3$) m/z: 433 (LZn(II) - 1 H), 454 (LZn(II)$NH_4$ - 2 H).

**Zn(II)-3:** SM (DIC, $NH_3$) m/z: 393 (LZn(II) - 1 H), 410 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 202 (78 700), 258 (74 100), 268 (46 700), 378 (4 600).

**Zn(II)-4 :** SM (DIC, $NH_3$) m/z: 463 (LZn(II) - 1 H), 482 (LZn(II)$NH_4$ - 2 H). UV/vis [dioxane/Tris·HCl 20 mM pH = 7,4 ; NaCl 150 mM (1/1, v/v)]: λ nm (ε $M^{-1}$ $cm^{-1}$) = 265 (69 200), 275 (45 700), 346 (5 800), 405 (6 100).

**Zn(II)-5 :** SM (DIC, $NH_3$) m/z: 401 (LZn(II) - 1 H),. UV/vis [$CH_3OH$/Tris·HCl 20 mM pH = 7,4 ; NaCl 150 mM (1/1, v/v)]: λ nm (ε $M^{-1}$ $cm^{-1}$) = 205 (43 300), 258 (50 500), 274 (30 900), 404 (3 000).

**Zn(II)-6:** SM (DIC, $NH_3$) m/z: 379 (LZn(II) - 1 H), 396 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 238 (31 100), 298 (26 200), 346 (10 500, épaulement), 473 (3 000).

**Zn(II)-7 :** SM (DIC, $NH_3$) m/z: 379 (LZn(II) - 1 H), 396 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 202 (55 500), 258 (63 600), 268 (43 400, épaulement), 374 (4 000).

**Zn(II)-8:** SM (DIC, $NH_3$) m/z: 449 (LZn(II) - 1 H), 466 (LZn(II)$NH_4$ - 2 H). UV/vis [dioxane/Tris·HCl 20 mM pH = 7,4 ; NaCl 150 mM (1/1, v/v)]: λ nm (ε $M^{-1}$ $cm^{-1}$) = 264 (57 800), 272 (43 600, épaulement), 390 (5 400).

**Zn(II)-9 :** SM (ES-SM, < 0) m/z: 735 (LZn(II)HCl). UV/vis [DMSO/Tris·HCl 20 mM pH = 7,4 ; NaCl 150 mM (8/2, v/v)]: λ nm (ε $M^{-1}$ $cm^{-1}$) = 276 (54 200), 340 (5 800), 352 (7 700), 397 (5 100).

**Zn(II)-10 :** SM (DIC, $NH_3$) m/z: 407 (LZn(II) - 1 H), 424 (LZn(II)$NH_4$ - 2 H). UV/vis [$CH_3OH$/Tris·HCl 20 mM pH = 7,4 ; NaCl 150 mM (1/1, v/v)]: λ nm (ε $M^{-1}$ $cm^{-1}$) = 202 (63 600), 260 (65 600), 368 (4 400).

**Zn(II)-12-Cl :** SM (DIC, $NH_3$) m/z: 370 (LZn(II)Cl). UV/vis ($CH_3OH$): λ nm (ε $M^{-1}$ $cm^{-1}$) = 288 (31 800), 299 (27 900), 369 (3 400), 490 (13 200).

**Zn(II)-13 :** UV/vis [$CH_3OH$/Tris·HCl 20 mM pH = 7,4; NaCl 150 mM (1/1, v/v)]: λ nm (ε $M^{-1}$ $cm^{-1}$) = 232 (55 400); 300 (9 600), 314 (9 200), 364 (700, épaulement).

**Zn(II)-14 :** UV/vis [$CH_3OH$/Tris·HCl 20 mM pH = 7,4; NaCl 150 mM (1/1, v/v)]: λ nm (ε $M^{-1}$ $cm^{-1}$) = 236 (43 900), 304 (8 700), 316 (8 000).

**Zn(II)-16:** SM (électrospray, > 0) m/z: 425 (LZn(II) - 1 H). UV/vis [$CH_3OH$/Tris·HCl 20 mM pH = 7,4; NaCl 150 mM (1/1, v/v)]: λ nm (ε $M^{-1}$ $cm^{-1}$) = 262 (44 700), 276 (22 300, épaulement), 308 (5 900), 390 (2 000).

**Zn(II)-17 :** SM (électrospray, > 0) m/z: 439 (LZn(II) - 1 H). UV/vis [$CH_3OH$/Tris·HCl 20 mM pH = 7,4; NaCl 150 mM (1/1, v/v)]: λ nm (ε $M^{-1}$ $cm^{-1}$) = 202 (66 900); 264 (92 500), 276 (47 100, épaulement), 318 (5 500), 398 (6 700).

**Zn(II)-18 :** SM (électrospray, > 0) m/z: 394 (LZn(II) - 1 H). UV/vis [DMSO/Tris·HCl 20 mM pH = 7,4; NaCl 150 mM (8/2, v/v)]: λ nm (ε $M^{-1}$ $cm^{-1}$) = 273 (27 400); 309 (23 800), 332 (22 000); 4307 (3 800).

**Zn(II)-23 :** UV/vis [$CH_3OH$/Tris·HCl 20 mM pH = 7,4; NaCl 150 mM (1/1, v/v)]: λ nm (ε $M^{-1}$ $cm^{-1}$) = 213 (47 000); 234 (24 800), 274 (25 600), 303 (10 900, épaulement), 330 (11 600), 348 (12 300), 363 (13 100).

***Estimation des constantes d'affinité des ligands pour les ions métalliques :***

Des solutions de ligand à étudier $L_s$, de chélateur en compétition $L_c$ et d'ion métallique M dans le rapport 1/1/1 sont analysées par spectrophotométrie UV-visible à 20°C. La concentration de chaque composart est de 15 μM. Les spectres d'absorption UV-visible des espèces $L_s$, $ML_s$, $L_c$ ou $ML_c$ et les analyses spectrophotométriques des compétitions sont réalisées en présence de tampon Tris·HCl 20 mM ; NaCl 150 mM (pH = 7,4) puisque c'est le solvant utilisé durant les essais de resolubilisation de $A\beta_{1-42}$ décrits par la suite. Un solvant organique ($CH_3OH$, dioxane ou DMSO) a été ajouté afin d'avoir une bonne solubilité de tous les ligands et de tous les complexes métalliques aux concentrations utilisées dans ces expériences. Des contrôles ont montré que tous les ligands et tous les complexes métalliques étudiés donnent des résultats en accord avec la loi de Beer-Lambert dans les conditions expérimentales utilisées.

Les chélateurs d'ions métalliques ($L_c$) utilisés durant les expériences de compétition ont été choisis à partir de "The National Institute of Standards and Technology Standard Reference Data base 46" NIST (Critically Selected Stability Constants of Metal Complexes Database, version 4.0, U. S. Department of Commerce) et L. G. Sillen, A. E. Martell Stability Constants of Metai-Ion complexes, The Chemical Society London Publication, 1971.

Les chélateurs compétiteurs $L_c$ ont été sélectionnés afin de disposer d'une gamme de constante de stabilité pour l'ion métallique M présentant des intervalles d'à peu près un ou deux log et aussi afin d'avoir la possibilité pour chaque expérience de compétition de quantifier une des espèces $L_s$, $ML_s$, $L_c$ ou $ML_c$ à une longueur d'onde du spectre UV-vis sans contamination des trois autres espèces (Figure 7). Les constantes de stabilité K = [ML]/([M][L]) choisies pour les chélateurs compétiteurs sont données pour 25 °C , une force ionique de 0.1-0.2 ν et un rapport molaire ion métallique/chélateur de 1/1 dans les tableaux 1 et 2. Lorsque plus d'un complexe métallique peut se former entre l'ion métallique et le chélateur, le chélateur de compétition a seulement été sélectionné si le log K de son complexe métallique avec un rapport ion métallique/chélateur de 1/1 est supérieur à ceux de tous les autres complexes possibles.

Le log K d'un chélateur pour un ion métallique varie avec le pH de la solution comme déterminé par les équations suivantes (C. S. Atwood et al., J. Neurochem. 2000, 75, 1219-1233 ; A. Ringblom, Complexation in Analytical Chemistry,

1963, Interscience, New York; G. Schwarznbach et al., Complexometric Titrations, 1969, Meuthuen, New York):

$$\log K_{app} = \log K - \log \alpha \qquad \text{(Eq. 1)}$$

où:

$$\alpha = [H^+]^n/(K_{a1} \times K_{a2} \times K_{a3} \times ... \times K_{an}) + [H^+]^{n-1}/(K_{a1} \times K_{a2} \times K_{a3} \times ... \times K_{an-1}) + ... + [H^+]/K_{a1} + 1 \qquad \text{(Eq. 2)}$$

$K_a = 10^x$, où $x = -pK_a$ du chélateur (les valeurs $K_a$ sont écrites dans un ordre décroissant des valeurs de $pK_a$), et n = no. de la valeur de $pK_a$. Les valeurs de $\log K_{app}$ pour les chélateurs compétiteurs utilisés à pH = 7,4 sont présentées dans les Tableaux 1 et 2.

Lorsque $L_s$, $L_c$ et M sont utilisés dans un rapport 1/1/1, et que $L_s$ forme avec l'ion métallique un complexe MLs et $L_c$ forme avec l'ion métallique un complexe $ML_c$

$$L_s + M \underset{}{\overset{K_s}{\rightleftharpoons}} ML_s \qquad K_s = \frac{[ML_s]}{[L_s]\ [M]} \qquad \text{(Eq. 3)}$$

$$L_c + M \underset{}{\overset{K_c}{\rightleftharpoons}} ML_c \qquad K_c = \frac{[ML_c]}{[L_c]\ [M]} \qquad \text{(Eq. 4)}$$

$K_c$ est la valeur de $K_{app}$ pour le chélateur en compétition $L_c$, déterminée avec les équations 1 et 2.

On peut en déduire qu'à l'équilibre :

$$\frac{K_s}{K_c} = \frac{[ML_s]\ [L_c]}{[ML_c]\ [L_s]} \qquad \text{(Eq. 5)}$$

et

$$K_s = K_c \frac{[ML_s]\ [L_c]}{[ML_c]\ [L_s]} \qquad \text{(Eq. 6)}$$

La concentration initiale C est la même pour $L_s$, $L_c$ et M. Si les complexes $ML_s$ et $ML_c$ sont observés en même temps sur les spectres UV-vis pour $\log K_c > 6$ on peut considérer que tout l'ion métallique est complexé sous les formes $ML_s$ ou $ML_c$ et qu'à l'équilibre :

$$[ML_s] + [ML_c] = C = [ML_s] + [L_s] = [ML_c] + [L_c]$$

La concentration $[ML_s]$ = x % de C étant mesurée sur les spectres UV-visible, on en déduit les concentrations $[L_s]$ = (1 - x) % de C, $[ML_c]$ = (1 - x) % de C et $[L_c]$ = x % de C et:

$$K_s = K_c \frac{x^2}{(x-1)^2} \qquad \text{(Eq. 7)}$$

Certaines expériences ont également été faites, dans les mêmes solvants, avec $L_s$ et M dans un rapport 1/1 (15 $\mu$M de chaque) mais avec différentes stoechiométries de $L_c$. Pour une concentration initiale C = $L_s$ = M, la concentration de ligand compétiteur [Lc] = y % de C qui, à l'équilibre, permet d'obtenir le rapport de concentration $[ML_s]/[L_s] = 1$ et donc pour laquelle les concentrations $[ML_s] = [L_s]$ = 50 % de C est déduite de graphes comme ceux obtenus sur la Figure 8. Dans ces conditions $[ML_s] = [ML_c]$; $[ML_c] + [L_c] = Y$, $y \geq 50$ et:

$$K_s = K_c \frac{y - 50}{50} \qquad \text{(Eq. 8)}$$

Lorsque log $K_s \leq 6$, ces équations ne sont pas utilisées car les pourcentages d'ion métallique libre en solution ou chélaté par le solvant ou le tampon ne sont pas négligeables.

Ainsi, dans le cas de **12** en présence de Zn(II), il a seulement été déterminé, dans le solvant étudié, le nombre d'équivalent de $ZnCl_2$ pour que 15 $\mu$M de ligand soit entièrement sous forme de complexe de Zn(II) dans le solvant étudié, le ligand étant en compétition avec le tampon Tris et le DMSO pour la complexation de l'ion métallique. Par comparaison avec d'autres chélateurs dans les mêmes conditions : 2, 2'-bipyridine (log $K_{app} \approx 5$ ; 250 eq $ZnCl_2$) < **12** (100 eq $ZnCl_2$)< 1,10-phénanthroline (log $K_{app}$ = 6,3 ; 50 eq $ZnCl_2$). D'où une estimation de log $K_s \approx 6$.

De la même façon, dans le tampon Tris/$CH_3OH$ (1/1, v/v), le nombre d'équivalent de Zn(II) est compris entre 100 et 200 eq pour **13,** entre 500 et 1000 eq pour **14,** entre 7,5 et 10 eq pour **18** et entre 100 et 200 eq pour **23.**

Les Tableaux 1 et 2 résument les valeurs observées lors de compétitions et le Tableau 3 présente les valeurs des constantes d'affinité qui en ont été déduites.

Le Tableau 4 met en évidence que log $K_s$ varie peu suivant la composition des différents milieux étudiés.

**Tableau 1:** Réaction de compétition entre les ligands ($L_s$), $CuCl_2$ (M) et un ligand compétiteur ($L_c$) dans un rapport 1/1/1 à 15 $\mu$M. Pour chaque ligand $L_s$, les solvants employés sont ceux décrits dans le Tableau 3. Le pourcentage de l'espèce $CuL_s$ à l'équilibre est donné. Les valeurs entre parenthèses correspondent au nombre d'équivalents de $L_c$ permettant la formation de 50 % de $CuL_s$ à partir d'un mélange de $L_s$ (15 $\mu$M, 1 équivalent) et $CuCl_2$ (1/1, mol/mol). $K_1$ est la constante de stabilité pour l'espèce $ML_c$. $K_2$ est la constante de stabilité pour l'espèce $M(L_c)_2$.

| | EDA | biPy | HIDA | Dien | EDDA | EDTA | Trien | CTDA | Tetren |
|---|---|---|---|---|---|---|---|---|---|
| log $K_1$ | 10,5 | 8,1 | 11,8 | 15,9 | 16,2 | 18,8 | 20,1 | 22,0 | 22,8 |
| log $K_1$ app | 7,8 | 8,1 | 10,5 | 11,8 | 13,9 | 15,9 | 16,0 | 17,1 | 17,9 |
| log $K_2$ | 9,1 | 5,5 | 4,0 | 5,0 | | | | | |
| **3** | 100 | 100 | 100 | - | 100 | 50 (1 eq) | 30 | 15 | 0 |
| **4** | - | - | 100 | - | 90 | 40 (0,65 eq) | - | - | - |
| **5** | - | - | - | - | - | 45 | - | - | - |
| **6** | - | - | - | - | - | 75 | - | - | - |
| **7** | - | - | - | - | - | 55 (1,1 eq) | - | - | - |
| **8** | - | - | - | - | - | 40 (0,65 eq) | - | - | - |
| **9** | - | - | - | 100 | - | 60 | 45 (0,75 eq) | - | 0 |
| **10** | - | - | - | - | - | 45 | - | - | - |
| **12** | - | - | - | - | - | 55 | - | - | - |
| **13** | - | - | - | - | 65 | 25 | - | - | - |
| **17** | - | - | - | - | - | 55 | - | - | - |

(suite)

| | EDA | biPy | HIDA | Dien | EDDA | EDTA | Trien | CTDA | Tetren |
|---|---|---|---|---|---|---|---|---|---|
| log $K_1$<br>log $K_1$ app<br>log $K_2$ | 10,5<br>7,8<br>9,1 | 8,1<br>8,1<br>5,5 | 11,8<br>10,5<br>4,0 | 15,9<br>11,8<br>5,0 | 16,2<br>13,9 | 18,8<br>15,9 | 20,1<br>16,0 | 22,0<br>17,1 | 22,8<br>17,9 |
| **23** | - | - | - | - | - | 30 | - | - | - |

**Tableau 2:** Réaction de compétition entre les ligands ($L_s$), $ZnCl_2$ (M) et un ligand compétiteur ($L_c$) dans un rapport 1/1/1 à 15 μM.

| | Trien | NTA | EGTA | EDTA | DTPA |
|---|---|---|---|---|---|
| log $K_1$<br>log $K_1$ app<br>log $K_2$ | 11,9<br>7,8 | 10,5<br>8,0<br>3,8 | 12,6<br>9,4 | 16,5<br>13,7 | 18,2<br>13,9 |
| **3** | 100 | 100 | 100 | 30 (0,7 eq) | 20 |
| **4** | - | - | - | 20 | - |
| **5** | - | - | - | 55 | - |
| **6** | - | - | - | 75 | - |
| **7** | - | - | - | 55 | - |
| **8** | - | - | - | 30 | - |
| **9** | 100 | 100 | - | 65 | - |
| **10** | - | - | - | 45 | - |
| **17** | - | - | - | 35 | - |

Pour chaque ligand $L_s$, les solvants sont ceux décrits dans le Tableau 3. Le pourcentage de l'espèce $ZnL_s$ à l'équilibre est donné. Les valeurs entre parenthèses correspondent au nombre d'équivalents de $L_c$ permettant la formation de 50 % de $ZnL_s$ à partir d'un mélange de $L_s$ (15 μM, 1 équivalent) et $ZnCl_2$ (1/1, mol/mol). $K_1$ est la constante de stabilité pour l'espèce $ML_c$. $K_2$ est la constante de stabilité pour l'espèce $M(L_c)_2$.

**Tableau 3 :** Espèces formées lors des réactions de titration avec $CuCl_2$ ou $ZnCl_2$ et constantes d'affinité des différents ligands pour les ions Cu(II) ou Zn(II) à pH = 7,4.

| Ligand | Complexe observé par titration avec $Cu^{II}$ (stoechiométrie L/Cu(II) observée) | log $KCu^{II}$ | Complexe observé par titration avec $Zn^{II}$ (stoechiométrie L/Zn(II) observée) | Log $KZn^{II}$ | Solvant |
|---|---|---|---|---|---|
| **3** | $LCu^{II}$ (1/1) | 15,9 ± 1[a] | $LZn^{II}$ (1/1) | 13,3 ± 1[a] | Tampon/$CH_3OH$ (1/1) |
| **4** | $LCu^{II}$ (1/1) | 15,4 ± 1[a] | $LZn^{II}$ (1/1) | 12,5 ± 1[b] | Tampon/dioxane[c] |
| **5** | $LCu^{II}$ (1/1) | 15,7 ± 1[b] | $LZn^{II}$ (1/1) | 13,9 ± 1[b] | Tampon/$CH_3OH$ (1/1) |
| **6** | $LCu^{II}$ (1/1) | 16,6+1[b] | $LZn^{II}$ (1/1) | 14,4 ± 1[b] | Tampon/$CH_3OH$ (1/1) |

(suite)

| Ligand | Complexe observé par titration avec $Cu^{II}$ (stoechiométrie L/Cu(II) observée) | log $KCu^{II}$ | Complexe observé par titration avec $Zn^{II}$ (stoechiométrie L/Zn(II) observée) | Log $KZn^{II}$ | Solvant |
|---|---|---|---|---|---|
| **7** | $LCu^{II}$ (1/1) | 16,0 ± 1 [a] | $LZn^{II}$ (1/1) | 13,9 ± 1 [b] | Tampon/$CH_3OH$ (1/1) |
| **8** | $LCu^{II}$ (1/1) | 15,4 ± 1 [a] | $LZn^{II}$ (1/1) | 13,0 ± 1 [b] | Tampon/dioxane [c] |
| **9** | $LCu^{II}$ (1/1) | 15,7 ± 1 [a] | $LZn^{II}$ (1/1) | 14,2 ± 1 [b] | Tampon/DMSO (2/8) |
| **10** | $LCu^{II}$ (1/1) | 15,7 ± 1 [b] | $LZn^{II}$ (1/1) | 13,5 ± 1 [b] | Tampon/$CH_3OH$ (1/1) |
| **12** | $LCu^{II}$ (1/1) | 16,0 ± 1 [b] | $LZn^{II}$ (1/1) | ≈ 6 | Tampon/DMSO (2/8) |
| **13** | $LCu^{II}$ (1/1) | 14,7 ± 1 [b] | - | - | Tampon/$CH_3OH$ (1/1) |
| **17** | $LCu^{II}$ (1/1) | 16,0 ± 1 [b] | $LZn^{II}$ (1/1) | 13,2 ± 1 [b] | Tampon/$CH_3OH$ (1/1) |
| **23** | $LCu^{II}$ (1/1) | 15,2 ± 1 [b] | - | - | Tampon/$CH_3OH$ (1/1) |

a) D'après l'équation 8.
b) D'après l'équation 7.
c) Dioxane/tampon Tris·HCl 20 mM pH = 7,4 ; NaCl 150 mM = 1/1 et 8/2 pour les expériences avec $CuCl_2$ et $ZnCl_2$, respectivement.

**Tableau 4:** Comparaison des réactions de compétition entre le ligand 3 ($L_s$), $CuCl_2$ ou $ZnCl_2$ (M) et le ligand compétiteur EDTA ($L_c$) dans un rapport 1/1/1 à 15 μM dans les différents solvants utilisés pour la détermination des constantes d'affinité apparentes pour les ions métalliques ($K_s$).

| Ion métallique | solvant | % $ML_s$ | $K_s$[a] |
|---|---|---|---|
| Cu(II) | Tampon/$CH_3OH$ (1/1) | 50 | 15,9 |
| | Tampon/dioxane (1/1) | 45 | 15,7 |
| | Tampon/DMSO (2/8) | 55 | 16,1 |
| Zn(II) | Tampon/$CH_3OH$ (1/1) | 30 | 13,0 |
| | Tampon/dioxane (2/8) | 33 | 13,1 |
| | Tampon/DMSO (2/8) | 35 | 13,1 |
| | $CH_3OH$ | 40 | 13,3 |

Le pourcentage de l'espèce $CuL_s$ à l'équilibre est indiqué.

a) D'après l'équation 7.

**Capacité des composés à augmenter la solubilité de protéines impliquées dans les maladies neurodégénératives :**

Dans le cerveau de personnes atteintes par la maladie d'Alzheimer, les plaques amyloïdes contiennent principalement un peptide (Aβ) comportant 39-43 acides aminés. Il est produit par la digestion de la Protéine Précurseur Amyloïde (APP) par les β- et γ-sécrétases. Les peptides $A\beta_{1-40}$ et $A\beta_{1-42}$ sont les plus nombreux. La pathogénèse est

liée à leur accumulation et plus particulièrement à celle de A$\beta_{1-42}$ qui est le plus amyloïdogénique et dont la production est amplifiée par les mutations induisant la maladie d'Alzheimer ou par les facteurs de risque de cette maladie (M. P. Mattson, Nature, 2004, 430, 631-639; M. Citron, Nature Rev. Neurosci. 2004, 5, 677-685).

***Réactifs pour les expériences réalisées en présence du peptide amyloïde A$\beta_{1-42}$:***

Avant utilisation, l'eau [qualité MilliQ (Millipore] et toutes les solutions tampons ont été traitées sur résine Chelex-100 (Biorad) (5 mg/mL) et filtrées à travers des filtres 0,2 μm (Whatman) pour enlever d'éventuelles traces d'ions métalliques ou de particules.

$CuCl_2$, $ZnCl_2$ ou $FeCl_3$, de qualité puriss p.a. proviennent de chez Fluka.

Le peptide β-amyloïde A$\beta_{1-42}$ (A$\beta_{1-42}$) a été synthétisé, purifié (jusqu'à une pureté supérieure à 95 %) et caractérisé par analyse sur HPLC et par spectrométrie de masse MALDI-TOF. Les solutions de travail du peptide ont été préparées en solubilisant 1 mg de peptide lyophilisé dans 500 μL d'eau et 500 μL d'une solution aqueuse de NaOH pH = 12,0 ; sous agitation dans un Thermomixer Comfort (Eppendorf). Les préparations de peptide sont ensuite centrifugées 10 min à 9 000 t/min et le surnageant est utilisé comme solution "stock de Aβ". La concentration en peptide du "stock de Aβ" est déterminée immédiatement par test colorimétrique avec un kit Micro BCA Protein Assays (Pierce) à partir de gammes étalon réalisées avec des quantités connues d'albumine de sérum bovin (BSA) puis la solution de peptide A$\beta_{1-42}$ est aliquotée et congelée rapidement dans de l'azote liquide avant d'être conservée à -20 °C jusqu'à son utilisation.

Les ligands sont employés sous la forme de chlorhydrates sauf dans le cas de **1** et **16** qui sont utilisés sous sa forme chlorhydrate obtenue en cours de synthèse, respectivement 2,2'-méthanediyl-bis(8-hydroxy-2-quinoléinium) dichlorure dihydrate et 2,2'-(méthanediyl)-bis(7-méthyloxy-8-hydroxy-2-quinoléinium) dichlorure. Les sels sont générés par addition d'un équivalent d'acide chlorhydrique par équivalent de fonction azotée du ligand dissout dans du DMSO. Après évaporation du solvant, ces sels sont dissouts à la concentration désirée avec du DMSO et conservés à -20 °C jusqu'à leur utilisation.

***Analyse de la précipitation de A$\beta_{1-42}$ en fonction du rapport Cu(II)/peptide:*** Le protocole a été établi à partir des travaux de : C. S. Atwood et al., J. Neurochem. 2000, 75, 1219-1233. Les concentrations finales sont indiquées. A$\beta_{1-42}$ (5 μM, 500 μL) est mis à agréger dans du tampon Tris·HCl 20 mM ; NaCl 150 mM (pH = 7,4) durant 2 heures à 37 °C sous agitation à 1400 t/min en présence de différentes stoechiométries de $CuCl_2$ dissout dans du DMSO (50 μL). Les échantillons (volume final = 550 μL) sont ensuite centrifugés durant 20 min à 9 000 t/min et 500 μL de surnageant sont prélevés. Le tube contenant le culot de précipitation reçoit alors 450 μL de mélange tampon d'expérience /DMSO (91/9, v/v). Puis la concentration en protéine est déterminée sur le surnageant et le culot par Micro BCA Protein Assays (Pierce): chaque échantillon reçoit un volume de révélateur et est incubé durant 1 heure à 60°C sous agitation à 1 400 t/min puis l'absorbance à 562 nm est mesurée. Les quantifications sont réalisées à partir de gammes étalon de BSA. Les quantités de A$\beta_{1-42}$ réellement contenues dans le culot et le surnageant sont obtenues après une correction due à la présence d'une partie résiduelle du surnageant (50/550 μL) dans la fraction contenant le culot. Pour toutes les expériences, le résultat de l'addition du pourcentage de A$\beta_{1-42}$ dans le surnageant avec le pourcentage de A$\beta_{1-42}$ dans le culot donne des valeurs proches de 100 %.

L'ion Cu(II) est connu pour induire le maximum de peptide-Aβ agrégé insoluble (C. S. Atwood et al., J. Biol. Chem. 1998, 273, 12817-12826). Le rapport Cu(II)/peptide induisant le maximum d'agrégation dans les conditions expérimentales mises en oeuvre a été tout d'abord déterminé. La Figure 9 résume les résultats obtenus. Sans addition de Cu(II), 47 % de A$\beta_{1-42}$ est précipité. L'agrégation augmente quand le rapport Cu(II)/A$\beta_{1-42}$ augmente jusqu'à 2,5 Cu(II) par A$\beta_{1-42}$ puis se stabilise (82 % de peptide agrégé).

***Inhibition de la précipitation de A$\beta_{1-42}$ par l'ajout de ligand :***

Le protocole a été établi à partir des travaux de : C. S. Atwood et al., J. Neurochem. 2000, 75, 1219-1233. Les concentrations finales sont indiquées. 500 μL de A$\beta_{1-42}$ (5 μM) est mis à agréger dans du tampon Tris·HCl 20 mM ; NaCl 150 mM (pH = 7,4) durant 1 heure à 37 °C sous agitation à 1400 t/min en l'absence ou en présence d'ions métalliques : $CuCl_2$, $ZnCl_2$ ou $FeCl_3$ (20 μM). Puis 50 μL de ligand à tester (200 μM) dissout dans du DMSO sont alors ajoutés et les échantillons sont incubés 1 heure de plus à 37 °C sous agitation à 1 400 t/min. Les échantillons sans ligand reçoivent aussi 50 μL de DMSO. Les échantillons (volume final = 550 μL) sont ensuite centrifugés durant 20 min à 9 000 t/min et 500 μL de surnageant sont prélevés. Le tube contenant le culot de précipitation reçoit alors 450 μL de mélange tampon d'expérience/DMSO (91/9, v/v). Puis la concentration en protéine est déterminée sur le surnageant et le culot par Micro BCA Protein Assays (Pierce): chaque échantillon reçoit un volume de révélateur et est incubé durant 1 heure à 60 °C sous agitation à 1 400 t/min puis l'absorbance à 562 nm est mesurée. Les quantifications sont réalisées à partir de gammes étalon de BSA. Les quantités de A$\beta_{1-42}$ réellement contenues dans le culot et le surnageant sont obtenues après une correction due à la présence d'une partie résiduelle du surnageant dans la fraction contenant le

culot. Dans le cas de **1, 2, 6, 12, 13, 14, 16, 17, 18, 23,** du 8-hydroxyquinaldine et 8-aminoquinoléine, qui présentent une absorption à 562 nm dans les conditions de dosage, des blancs sont réalisés en présence du ligand testé et de l'ion métallique étudié. Pour toutes les expériences, le résultat de l'addition du pourcentage de $A\beta_{1-42}$ dans le surnageant avec le pourcentage de $A\beta_{1-42}$ dans le culot donne des valeurs proches de 100 %.

Les résultats obtenus sont résumés dans le Tableau 5. Ils montrent la capacité des composés étudiés à augmenter la solubilité de $A\beta_{1-42}$ en présence d'ions métalliques et même, en particulier pour **1, 2, 12, 13, 14, 15, 17, 18, 23** et **24,** en absence de ces ions.

Un test supplémentaire (non présenté) a mis en évidence qu'en présence de $CuCl_2$, le taux de précipitation maximal du peptide était atteint au bout d'une heure d'incubation. On peut donc proposer que tout au moins dans le cas de l'agrégation du peptide en présence de $CuCl_2$, les ligands (qui sont rajoutés seulement après 1 heure d'incubation) ne font pas qu'empêcher l'agrégation du peptide mais peuvent probablement agir sur des agrégats préalablement formés.

**Tableau 5:** Analyse de la solubilisation par les différents ligands de $A\beta_{1-42}$ agrégé en absence ou en présence d'ions métalliques.

| | sans métal % Soluble Aβ | $CuCl_2$ % Aβ soluble | $ZnCl_2$ % Aβ soluble | $FeCl_3$ % Aβ soluble |
|---|---|---|---|---|
| Aβ seul | 55 ± 4 | 18 ± 2 | 29 ± 3 | 47 |
| **1** | 63 ± 4 | 60 ± 5 | 63 | 61 |
| **2** | 66 | 45 ± 5 | 57 | 58 |
| **3** | 56 | 47 ± 3 | 59 | 60 |
| **4** | 55 | 30 | 49 | 45 |
| **5** | 60 | 51 ±1 | 50 | 52 |
| **6** | 56 | 58 ± 3 | 54 | 51 |
| **7** | 59 | 35 | 60 | 40 |
| **8** | 59 | 36 | 58 | 41 |
| **9** | 36 | 19 | 47 | 38 |
| **10** | 52 | 42 | 43 | 43 |
| **11** | 48 | 48 | 45 | 43 |
| **12** | 69 | 69 ± 2 | 52 | 59 |
| **13** | 71 ± 2 | 55 ± 3 | 57 | 66 |
| **14** | 79 ± 3 | 45 ± 3 | 57 | 51 |
| **15** | 72 | 35 | 23 | 42 |
| **16** | 53 | 48 ± 3 | 50 | 44 ± 6 |
| **17** | 63 ± 3 | 38 ± 4 | 42 ± 4 | 38 |
| **18** | 66 | 46 ± 3 | 62 ± 5 | 49 |
| **23** | 73 | 60 ± 4 | 58 ± 4 | 62 |
| **24** | 64 | 21 | 21 | 38 |
| 8-hydroxyquinoléine | 85 | 70 ± 3 | 88 | 80 |
| 8-hydroxyquinaldine | 69 | 52 | 62 | 51 |
| Clioquinol | 63 | 55 ± 3 | 37 | 51 |
| 8-aminoquinoléine | 66 ± 3 | 40 | 67 | 58 |
| EDTA | 41 | 65 | - | - |

$A\beta_{1-42}$ (5 μM) est incubé 1 heure à 37 °C dans du tampon 20 mMTris·HCl (pH = 7,4), 150 mM NaCl en présence ou en absence de 20 μM de sel métallique ($CuCl_2$, $ZnCl_2$ ou $FeCl_3$) puis 1 heure de plus en présence de 200 μM ligand puis

les mélanges réactionnels sont centrifugés. Les quantités de A$\beta_{1-42}$ soluble et précipité sont déterminées dans le surnageant et le précipité, respectivement, à l'aide d'un kit Micro BCA Protein Assays.

Des expériences ont aussi été réalisées pour A$\beta_{1-42}$ (5 $\mu$M), $CuCl_2$ (12,5 $\mu$M) et le ligand (12,5 $\mu$M) afin d'analyser l'effet, sur la réaction d'agrégation, d'une stoechiométrie minimale (1/1) en ligand par rapport à l'ion métallique pour une concentration en cet ion entraînant un maximum de précipitation du peptide A$\beta_{1-42}$. Pour toutes les expériences, le résultat de l'addition du pourcentage de A$\beta_{1-42}$ dans le surnageant avec le pourcentage de A$\beta_{1-42}$ dans le culot donne des valeurs proches de 100 %.

Les résultats obtenus sont résumés sur la Figure 10.

Une stoechiométrie de 2,5 équivalents de Cu(II) par peptide a été choisie car c'est la valeur minimale induisant le maximum d'agrégation dans les conditions expérimentales employées (Figure 9). Pour les expériences contrôles avec des dérivés comportant un seul résidu quinoléine, 5 équivalents de ligand ont été ajoutés car des complexes de type $L_2Cu$ sont classiquement invoqués dans leur cas.

La Figure 10 résume les résultats observés en les comparant à ceux des expériences précédentes. Remarquer que l'activité de **1, 3, 5, 6, 11** et **18** est statistiquement identique si on considère les valeurs avec un écart type $\leq$ 5 %, quelles que soient les conditions employées, alors que celle de leurs analogues comportant un seul résidu quinoléine (8-hydroxyquinoléine, clioquinol et 8-hydroxyquinaldine) diminue quand l'excès par rapport au peptide ou à Cu(II) diminue. Pour ces deux conditions expérimentales, les valeurs sont également voisines pour **13** et **14** Dans ces deux conditions expérimentales, **23** est également plus actif que son analogue comportant un seul résidu quinoléine (8-aminoquinoléine).

**Capacité des composés à diminuer le stress oxydatif :**

Il a été proposé que les interactions de A$\beta$ avec les ions Fe et Cu peuvent contribuer à la création des lésions observées dans les cerveaux atteints de la maladie d'Alzheimer. Les peptides A$\beta$ synthétiques exercent une toxicité qui corrèle avec une production de peroxyde d'hydrogène ($H_2O_2$) à partir de $O_2$ par l'intermédiaire d'un mécanisme d'oxydoréduction au niveau de l'ion métallique (M. P. Mattson, Nature 2004, 430, 631-639).

*Dosage du peroxyde d'hydrogène produit par A$\beta_{1-42}$ en présence de* ***$CuCl_2$, de réducteur, d'air et de ligand:*** Le protocole a été établi à partir des travaux de : X. Huang et al., J. Biol. Chem. 1999, 274, 37111-37116 ; X. Huang et al., Biochemistry 1999, 38, 7609-7616 ; C. Opazo et al., J. Biol. Chem. 2002, 277, 40302-40308; K. J. Barnham et al., J. Biol. Chem. 2003, 278, 42959-42965; G. D. Ciccotosto et al., J. Biol. Chem. 2004, 279, 42528-42534.

Les concentrations finales sont indiquées. Les réactions ont été réalisées dans le noir dans du tampon phosphate de sodium, 50 mM (pH = 7,4). A$\beta_{1-42}$ (0,2 $\mu$M) et $CuCl_2$ (0,4 $\mu$M) sont préincubés durant 1 heure à 37°C sous agitation à 1 400 t/min dans 375 $\mu$L de tampon. Puis 2 $\mu$L d'une solution de ligand à étudier (0,2; 0,4 ou 0,8 $\mu$M) dans du DMSO sont alors ajoutés. Après incubation durant une heure supplémentaire, 2 $\mu$L d'une solution aqueuse d'ascorbate de sodium (10 $\mu$M) sont ajoutés et l'incubation est poursuivie durant 5 min toujours sous agitation. La quantité de $H_2O_2$ produite est alors quantifiée à l'aide d'un kit Amplex Red $H_2O_2$/HRP Assay Kit (Molecular Probes) en ajoutant un volume de réactif de dosage sur les échantillons puis en les incubant durant 1 heure à 37 ° C sous agitation à 500 t/min. Les détections de $H_2O_2$ sont alors réalisées spectro-photométriquement à 563 nm. Les quantifications sont réalisées à partir de courbes étalons obtenues avec de quantités connues de $H_2O_2$. Les résultats obtenus sont résumés dans le Tableau 6 et sur la Figure 11.

Des expériences ont aussi été réalisées en présence d'une quantité connue de $H_2O_2$, ajoutée juste avant ou juste après l'addition d'ascorbate de sodium, afin d'étudier la stabilité de $H_2O_2$ dans les conditions expérimentales utilisées. Les résultats obtenus sont résumés dans le Tableau 7.

D'autres expériences ont été réalisées en présence de différentes concentrations de peptide A$\beta_{1-42}$ (0,2 à 0,53 $\mu$M) et $CuCl_2$ (0,4 $\mu$M), avec ou sans addition de ligand (0,4 $\mu$M) et de quantités connues de $H_2O_2$, afin d'étudier l'influence de la stoechiométrie A$\beta_{1-42}$/Cu sur la production de $H_2O_2$ par les systèmes étudiés. Les résultats obtenus sont résumés sur la Figure 11.

Le peptide A$\beta$ peut chélater différentes stoechiométries de Cu(II) et ainsi former différents types de complexes. Toutes ces espèces peuvent avoir différentes capacités à produire $H_2O_2$ en présence de dioxygène et de réducteur. La titration de la quantité de $H_2O_2$ produite par 0,4 $\mu$M de Cu(II) en fonction de la concentration de A$\beta_{1-42}$ fait apparaître ce phénomène (Figure 11). La quantité de $H_2O_2$ produite augmente quand le rapport Cu/A$\beta_{1-42}$ augmente. Les mêmes expériences ont été réalisées en présence de 1 équivalent de ligand 3 ou 7 par Cu(II). Les deux composées inhibent pratiquement toute la production de $H_2O_2$ quel que soit le rapport Cu/ A$\beta_{1-42}$ testé.

Le Tableau 6 montre que les différents dérivés de quinoléine testés inhibent la production de $H_2O_2$ quelles que soient les conditions expérimentales utilisées.

**Tableau 6:** Comparaison de l'inhibition par les différents ligands (0,4 μM) de la production de $H_2O_2$ réalisée par $A\beta_{1-42}$ (0,2 et 0,5 μM) en présence de $CuCl_2$ (0,4 μM), d' ascorbate (10 μM) et d'air.

| Ligand 1 eq/Cu(II) (quand non précisé) | sans $A\beta_{1-42}$ | 0,2 μM $A\beta_{1-42}$ Cu/Aβ = 2 | 0,5 μM $A\beta_{1-42}$ Cu/Aβ = 0,75 |
|---|---|---|---|
| sans ligand | 2,91 ± 0,22 | 2,83 ± 0,19 | 1,08 |
| **1** | 0,54 | 0,75 ± 0,11 | 0,76 |
| **2** | 0,56 | 0,80 ± 0,23 | 0,61 |
| **3** 0,5 eq/Cu(II)<br>1 eq/Cu(II)<br>1,5 eq/Cu(II) | 2,12<br>0,84<br>- | 0,94<br>0,71 ± 0.02<br>0,49 | -<br>0,55<br>- |
| **4** | 0,12 | 0,37 ± 0,09 | 0,35 |
| **5** | 0,39 | 0,55 ± 0,08 | - |
| **6** | 0,82 | 0,86 ± 0,11 | - |
| **7** | 0,75 | 0,89 ± 0,07 | 0,68 |
| **8** | 0,66 | 0,89 ± 0,09 | 0,64 |
| **9** | 0,66 | 0,82 ± 0,08 | 0,82 |
| **12** | 0,40 | 0,47 ± 0,09 | - |
| **13** | 0,89 | 2,10 | 0,70 |
| **14** | 0,74 | 1,99 | 0,78 |
| **15** | - | 1,02 | - |
| **16** | - | 0,70 | - |
| **17** | - | 0,69 | - |
| **18** | 0,47 | 0,47 | 0,45 |
| **23** | 0,65 | 0,69 | 0,49 |
| **24** | 0,49 | 0,62 | 0,47 |
| 8-hydroxyquinoléine<br>1 eq/Cu(II)<br>2 eq/Cu(II) | <br>2,02<br>0,62 | <br>0,69<br>0,71 ± 0,05 | <br>0,75<br>0,62 |
| Clioquinol<br>1 eq/Cu(II)<br>2 eq/Cu(II) | <br>2,07<br>0,54 | <br>0,89 ± 0,08<br>0,78 ± 0,06 | <br>0,64<br>0,57 |
| sans ligand et sans Cu(II) | 0,49 ± 0,19 | 0,69 | 0,51 |
| sans ligand, sans ascorbate et sans Cu(II) | 0 | 0,06 | - |

Les chiffres de tableau correspondent à la quantité d'$H_2O_2$ (exprimé en nanomoles) dosée à l'aide d'un kit Amplex Red $H_2O_2$/HRP Assay.

Dans une expérience contrôle faite avec uniquement le ligand sans peptide amyloïde $\beta_{1-42}$ et sans Cu, la détermination de la production de $H_2O_2$ due à des traces métalliques présentes dans le milieu d'étude est toujours inférieure à 0,49 ± 19 nanomoles d'$H_2O_2$.

Le Tableau 7 montre que, dans les conditions expérimentales utilisées, les ligands peuvent être divisés en deux catégories : (i) ceux qui inhibent la production de $H_2O_2$ mais ne le dégradent pas (toute la quantité de $H_2O_2$ ajoutée est retrouvée) ; (ii) ceux pour lesquels la diminution de $H_2O_2$ est associée à sa dégradation (comme dans le cas de 12 où toute la quantité de $H_2O_2$ ajoutée n'est pas retrouvée).

**Tableau 7:** Analyse de la stabilité de $H_2O_2$ au cours des expériences d'inhibition par les ligands de la production de $H_2O_2$ réalisée par $A\beta_{1-42}$ (0,2 ou 0,4 $\mu$M) en présence de $CuCl_2$ (0,4 $\mu$M), d'ascorbate (10 $\mu$M) et d'air.

| $A\beta_{1-42}$ 0.2 $\mu$M [a] 0,075 nanomole | Cu(II) 0.4 $\mu$M 0,15 nanomole | ascorbate 10 $\mu$M 3,8 nanomoles | Ligand 0,4 $\mu$M [a] 0,15 nanomole | $H_2O_2$ ajouté nanomoles | $H_2O_2$ dosé nanomoles |
|---|---|---|---|---|---|
| - | - | + | - | - | 0,49 ± 0,19 |
| - | - | + | - | 3,0 | 3,2 (3,5) |
| - | + | + | - | - | 2,91 ± 0.22 |
| - | + | + | - | 1,5 | 4,0 (4.4) |
| - | + | + | - | 1,5 [b] | 3,6 (4,4) |
| + | + | + | - | - | 2,83 ± 0,19 |
| + | + | + | - | 1,5 | 4,2 (4,3) |
| + | + | + | - | 1,5 [b] | 4,2 (4,3) |
| 0,4 $\mu$M | + | + | - | - | 1,6 |
| 0,4 $\mu$M | + | + | - | 1,5 | 3,0 (3,1) |
| 0,4 $\mu$M | + | + | - | 1,5 [b] | 3,0 (3,1) |
| - | + | - | **3** | 3,0 | 3,1 |
| - | + | + | **3** | - | 0,84 |
| - | + | + | **3** | 3,0 | 3,8 (3,8) |
| + | + | + | **3** | - | 0,71 ± 0,02 |
| + | + | + | **3** | 3,0 | 3,7 (3,7) |
| + | + | + | **3** | 3,0 [b] | 3,8 (3,7) |
| + | + | + | **4** | - | 0,37 ± 0,09 |
| + | + | + | **4** | 3,0 | 3,3 (3,4) |
| + | + | + | **5** | - | 0,55 ± 0,08 |
| + | + | + | **5** | 3,0 | 3,4 (3,5) |
| + | + | + | **6** | - | 0,87 ± 0,11 |
| + | + | + | **6** | 3,0 | 3,6 (3,9) |
| + | + | + | **12** | - | 0,47 ± 0,09 |
| + | + | + | **12** | 3,0 | 1,7(3,4) |
| + | + | + | **14** | - | 1,99 ± 0,11 |
| + | + | + | **14** | 3,0 [b] | 4,9 (5,0) |
| - | + | + | **18** | - | 0,47 |
| - | + | + | **18** | 3,0 [b] | 3,5 (3,5) |

L'analyse est obtenue en comparant les résultats obtenus avec ou sans addition d'une quantité connue de $H_2O_2$ dans le milieu réactionnel juste après l'addition d'ascorbate si non précisé par b). La valeur théorique en $H_2O_2$ s'il n'y a pas de dégradation est indiquée entre parenthèses. Elle correspond à l'addition du nombre de nanomoles de $H_2O_2$ ajoutés à ceux produits dans une réaction réalisée dans les mêmes conditions expérimentales mais sans ajout de $H_2O_2$. $H_2O_2$ est dosé à l'aide d'un kit AmplexRed $H_2O_2$/HRP Assay.

a) Quand non précisé
b) $H_2O_2$ ajouté juste avant l'ascorbate.

**Modulation de l'hydrophobicité :**

***Détermination des log $D_{7,4}$ :*** La méthode est adaptée de Z.-P Zhuang et al., J. Med. Chem., 2001, 44, 1905-1914. Le ligand (2,0 mg) est dissout dans 2 mL de 1-octanol puis 2 mL de tampon Tris·HCl 20 mM, pH = 7,4 ;

NaCl 150 mM sont ajoutés. Après un mélange au vortex durant 3 min à température ambiante suivi d'une centrifugation durant 5 min à 9 000 t/min, la concentration du ligand dans chaque phase est déterminée par spectrophotométrie UV-visible. Les fractions 1-octanol sont repartitionnées ainsi jusqu'à ce que des valeurs reproductibles de coefficient de partition soient obtenues. Ce coefficient est exprimé comme le logarithme décimal de [(la concentration de ligand contenu dans la phase 1-octanol)/(la concentration de ligand contenu dans la phase aqueuse tamponnée)] = log $D_{7,4}$. Les mesures ont été réalisées trois fois.

Le tableau 8 résume les résultats obtenus.

La valeur de log $D_{7,4}$ reflète l'hydrophobicité et donc la lipophilicité des composés qui est un des paramètres employés (avec d'autres tels le poids moléculaire) pour estimer les possibilités de biodistribution des molécules (H. van de Waterbeemd et al., Nature Rev. Drug Discovery 2003, 2, 192-204).

Pour des substitutions identiques des macrocycles aromatiques, les dérivés comportant un seul résidu quinoléine sont plus hydrophiles que ceux en comportant plusieurs. L'hydrophobicité peut être modulée par la longueur du bras de jonction entre les résidus quinoléine (comparer **7** et **10**), par la nature dudit bras (comparer **7** et **18**) et par la substitution des hydrogènes des cycles (comparer respectivement : **1** et **2 ; 3** et **4 ; 7,8, 9** et **17**) ou des bras de jonction (comparer **1, 3, 5** et **6**) par différents groupements.

**Tableau 8:** Valeur du logarithme décimal du coefficient de partage des ligands entre du 1-octanol (phase hydrophobe) et du tampon Tris·HCl 20 mM ; NaCl 150 mM (pH = 7.4) (phase hydrophile) (1/1, v/v).

| Ligand | PM | log $D_{7,4}$ |
|---|---|---|
| **1** | 301 | 3,3 ± 0,1 |
| **2** | 370 | 3,5 ± 0,1 |
| **3** | 330 | 4,4 ± 0,1 |
| **4** | 398 | 4,9 ± 0,1 |
| **5** | 338 | 3,8 ± 0,1 |
| **6** | 316 | 3,9 ± 0,1 |
| **7** | 316 | 3,5 ± 0,1 |
| **8** | 384 | 4,2 ± 0,1 |
| **9** | 636 | 5,6 ± 0,5 |
| **10** | 344 | 3,8 ± 0,1 |
| **11** | 474 | 3,7 ± 0,1 |
| **12** | 271 | 3,5 ± 0,1 |
| **13** | 328 | 2,7 ± 0,1 |
| **14** | 342 | 1,8 ± 0,1 |
| **15** | 272 | 2,0 ± 0,1 |
| **17** | 376 | 3,5 ± 0,1 |
| **18** | 331 | 3,0 ± 0,1 |
| **23** | 357 | 2,5 ± 0,1 |
| **24** | 299 | 2,6 ± 0,1 |
| 8-hydroxyquinoléine | 145 | 2,1 ± 0,1 |
| 8-hydroxyquinaldine | 159 | 2,4 ± 0,1 |
| Clioquinol | 305 | 3,8 ± 0,1 |
| 8-aminoquinoléine | 144 | 1,9 ± 0,1 |

**Analyse de la génotoxicité potentielle :**

***Test de AMES :*** La méthode est adaptée de D.M. Maron et B. N. Ames, Mutat. Res. 1983, 113, 173-215 ; D. E. Levin et al., Mutat. Res. 1982, 94, 315-330; D. E. Levin et al. Proc. Natl. USA 1982, 79, 7445-7449.

Deux lignées test (TA98 et TA100) de *Salmonella tiphimurium* mutant ($His^-$) ont été utilisées de par leur déficience à synthétiser l'histidine et de la confirmation des caractéristiques de ces lignées par des tests de marqueurs génétiques. Le test consiste en l'évaluation du potentiel des molécules testées à induire une mutation réverse au locus histidine de ces lignées. Ces deux lignées ont été fournies par le Dr. Bruce N. Ames (University of California, Berkeley, USA). Elles sont résistantes à l'Ampicilline et sensibles à la Tetracycline. Elles ne sont pas mutantes pour rfa, UvyB et UvyA. La lignée TA98 (his D3052) contient une mutation de phase de lecture (GC) tandis que la lignée TA100 (his G46) conteint une substitution de paire de base (GC). Le nombre de révertant spontané est de 37 $\pm$ 6 et 166 $\pm$ 15 colonies par plaques pour TA98 et TA100, respectivement.

Les composés à tester ont été dissous dans 100 % de DMSO (le solvant) et dilués logarithmiquement (d'un facteur 10) de façon à obtenir 4 concentrations test de 30 000 ; 3 000 ; 300 et 30 $\mu$g/mL Chaque série de 0,1 mL de solution stock à tester, 0,1 mL de lignée test et de milieu de culture avec ou pas 0,5 mL d'homogénat enzymatique (S9) de microsome de foie de rat ont été mélangés avec 2 mL d'agarose fondue (contenant 0,5 mM d'histidine et 0,5 mM de biotine). Ce mélange a été déposé à la surface d'une plaque de milieu agarose glucose minimal (30 mL pour chaque boite de pétrie) de façon à obtenir des concentrations finales en composé à tester de 3 000; 300; 30 et 3 $\mu$g/plaque. Les plaques ont alors été incubées à 37°C durant 48 heures. Les cultures ont été traitées ou non en présence d'une activation métabolique exogène par l'ajout de 0,5 mL de mélange S9 qui contenait 8 mM $MgCl_2$, 33 mM KCl, 4 mM NADP, 5 mM glucose-6-phosphate, 100 mM $NaH_2PO_4$ (pH = 7,4) et 4 % (v/v) d'homogénat enzymatique de microsome de foie de rat induit par Acrolor 1254 (S9). Les colonies révertantes des lignées test ont été dénombrées avec un compteur de colonies (Sigma). Les résultats ont été considérés comme significatifs uniquement si les valeurs obtenues pour le solvant seul et des composés de référence étaient dans une fourchette précédemment établie. Un nombre de colonies $\geq$ 3 fois au nombre de colonies observé pour le solvant seul est considéré comme mutagène de façon significative. Un composé induisant un nombre de colonie < 50 % de celles obtenues pour le solvant seul a été considéré comme toxique pour les bactéries testées. Tous les essais ont été réalisés en triplicate.

L'obtention de résultats attendus avec ces lignées pour 4-NPD, l'azide de sodium, 2-Anthramine et 2-Aminofluorène a permis de confirmer la bonne qualité de l'étude.

Les résultats obtenus sont résumés dans le tableau 9.

**Tableau 9 :** Résultats du test de Ames de mutagénicité de Salmonella sur les souches TA98 et TA100 de salmonelle en absence ou en présence d'homogénat enzymatique (S9) de microsome de foie de rat.

| Composé (valeur par plaque) | Ajout de S9 | TA98 | | TA100 | |
|---|---|---|---|---|---|
| | | Effet mutagène | Cytotoxicité | Effet mutagène | Cytotoxicité |
| DMSO (solvant) | non | 100 $\mu$L | > 100 $\mu$L | > 100 $\mu$L | > 100 $\mu$L |
| | oui | > 100 $\mu$L | > 100 $\mu$L | > 100 $\mu$L | > 100 $\mu$L |
| Clioquinol | non | - | 300 $\mu$g | - | 30 $\mu$g |
| | oui | - | 300 $\mu$g | - | 30 $\mu$g |
| **3** | non | > 3000 $\mu$g | > 3000 $\mu$g | > 3000 $\mu$g | > 3000 $\mu$g |
| | oui | > 3000 $\mu$g | > 3000 $\mu$g | > 3000 $\mu$g | > 3000 $\mu$g |
| **5** | non | > 3000 $\mu$g | > 3000 $\mu$g | - | 300 $\mu$g |
| | oui | > 3000 $\mu$g | > 3000 $\mu$g | > 3000 $\mu$g | > 3000 $\mu$g |
| **12** | non | > 3000 $\mu$g | > 3000 $\mu$g | > 3000 $\mu$g | > 3000 $\mu$g |
| | oui | > 3000 $\mu$g | > 3000 $\mu$g | > 3000 $\mu$g | > 3000 $\mu$g |

Un effet bactéricide du Clioquinol est observé sur les deux lignées. Aucun effet mutagène n'a été observé pour les composés polyquinoléine testés même pour les plus fortes doses étudiées. Aucun effet bactéricide n'a été observé pour ces composés même pour les plus fortes doses testées à l'exception du composé 5 à partir de 300 $\mu$g/plaque, sur la lignée TA100 en absence de S9.

## Revendications

1. Composé de formule (I) :

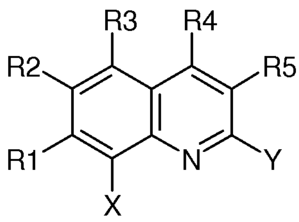

(I)

tels que dans la formule (I)
X représente un groupe -NRR', et
Y représente un groupe de formule :

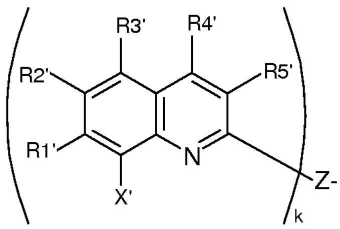

(IY)

dans laquelle X' représente un groupe -NRR', et
Z représente -NR"-,
où R"= alkyle éventuellement substitué par un ou plusieurs groupes choisis parmi OR, NRR', et
R représente un atome d'hydrogène ou un groupe alkyle ;
R'=H ou alkyle ;
R1, R2, R3, R4, R5, R1', R2', R3', R4' et R5', identiques ou différents, représentent indépendamment un atome choisi parmi H ou Halogène,
k représente 1 ;
ainsi que leurs stéréoisomères ou leur mélanges, leurs formes tautomères, leurs hydrates, solvates, leurs sels, formes libres et esters pharmaceutiquement acceptables.

**2.** Composé selon la revendication 1 choisi parmi :

N-Butyl-2,2'-imino-bis(8-quinoléinamine)
ainsi que leurs stéréoisomères ou leur mélanges, leurs formes tautomères, leurs hydrates, solvates, leurs sels, formes libres et esters pharmaceutiquement acceptables.

**3.** Composition pharmaceutique comprenant un composé de formule (I)

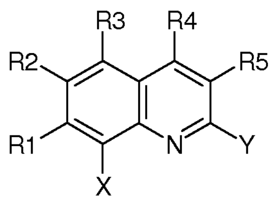

(I)

tel que défini en revendication 1 ou 2.

4. Utilisation d'un composé de formule (I) tel que défini en revendication 1 ou 2 pour la préparation d'un médicament pour prévenir et/ou traiter les maladies affectant le système nerveux central, telles que les maladies neurodégénératives.

5. Utilisation selon la revendication 4 telle que les maladies neurodégénératives sont choisies parmi la maladie d'Alzheimer, la maladie de Parkinson, la maladie d'Huntington, la sclérose latérale amyotrophique ou la trisomie 21, les encéphalopathies spongiformes, telle que la maladie de Kreutzfeld-Jacob.

6. Utilisation d'un composés tel que défini selon la revendication 1 ou 2 pour la préparation d'un médicament pour chélater les ions métalliques et/ou pour dissoudre les agrégats amyloïdes.

7. Utilisation selon la revendication 6 telle que lesdits métaux sont choisis parmi le cuivre et/ou fer.

8. Procédé de préparation d'un composé selon l'une quelconque des revendications 1 à 2 comprenant l'étape de couplage des composés de formule (VVII) et (VVII') :

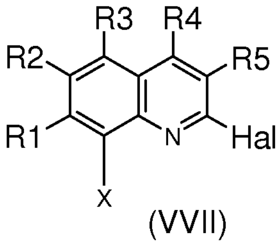

(VVII)

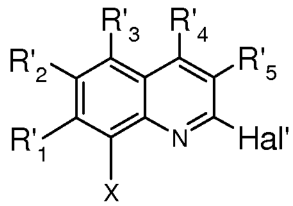

(VVII')

dans lesquelles Hal et Hal' sont des atomes d'halogène en présence d'un groupe de formule $-RNH_2$, éventuellement suivi de dérivatisation(s) du produit de formule (I) obtenu pour obtenir le produit de formule (I) souhaité et/ou de l'étape d'isolation du produit final obtenu.

**Patentansprüche**

1. Verbindung der Formel (I):

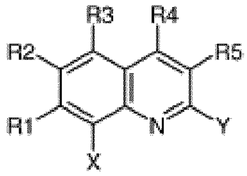

(I)

wie in Formel (I)
stellt X eine Gruppe -NRR' dar, und
Y stellt eine Gruppe der Formel:

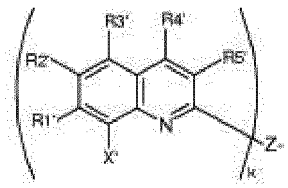

(IY)

dar,
worin X' eine Gruppe -NRR' darstellt, und
Z -NR"- darstellt,
wobei R" = Alkyl, gegebenenfalls substituiert durch eine oder mehrere Gruppe/n, die aus OR, NRR' ausgewählt ist/sind,
und
R ein Wasserstoffatom oder eine Alkylgruppe darstellt;
R' = H oder Alkyl;
R1, R2, R3, R4, R5, R1', R2', R3', R4' und R5', identisch oder verschieden, unabhängig ein Atom darstellen, das aus H oder Halogen ausgewählt ist,
k stellt 1 dar;
sowie deren Stereoisomere oder deren Gemische, deren tautomeren Formen, deren Hydrate, Solvate, deren Salze, freien Formen und Ester in pharmazeutisch geeigneter Form.

**2.** Verbindung nach Anspruch 1, ausgewählt aus:

N-Butyl-2,2'-imino-bis(8-chinolinamin)
sowie deren Stereoisomere oder deren Gemische, deren tautomeren Formen,
deren Hydrate, Solvate, deren Salze, freien Formen und Ester in pharmazeutisch geeigneter Form.

**3.** Pharmazeutische Zusammensetzung, eine Verbindung der Formel (I)

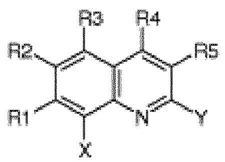

(I)

wie in Anspruch 1 oder 2 definiert umfassend.

**4.** Verwendung einer wie in Anspruch 1 oder 2 definierten Verbindung der Formel (I) zur Herstellung eines Medikaments zur Vorbeugung und/oder Behandlung von Krankheiten wie neurodegenerative Krankheiten, die sich auf das zentrale Nervensystem auswirken.

**5.** Verwendung nach Anspruch 4, wobei die neurodegenerativen Krankheiten aus Alzheimer, Parkinson, Huntington-Krankheit, amyotropher Lateralsklerose oder Trisomie 21, spongiformen Enzephalopathien wie etwa Kreutzfeld-Jacob ausgewählt sind.

**6.** Verwendung von wie in Anspruch 1 oder 2 definierten Verbindungen zur Herstellung eines Medikaments zum Chelatieren von Metallionen und/oder zum Auflösen von Amyloidaggregaten.

**7.** Verwendung nach Anspruch 6, wobei die Metalle aus Kupfer und/oder Eisen ausgewählt sind.

**8.** Verfahren zum Herstellen einer Verbindung nach einem der Ansprüche 1 bis 2, den Schritt einer Kopplung der Verbindungen der Formel (VVII) und (VVII'):

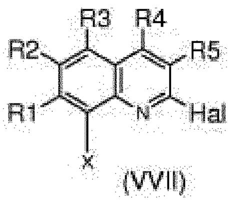

(VVII)

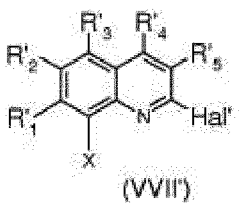

(VVII')

worin Hal und Hal' Halogenatome im Beisein einer Gruppe der Formel $-RNH_2$ sind, gegebenenfalls gefolgt von einer

oder mehreren Derivatisierung/en des erhaltenen Produkts von Formel (I), um das gewünschte Produkt von Formel (I) zu erhalten, und/oder den Isolationsschritt des erhaltenen Endprodukts umfassend.

## Claims

**1.** A compound of formula (I) :

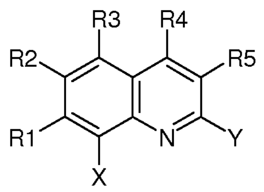

(I)

wherein in formula (I) :

X represents an -NRR' group and
Y represents a group of formula :

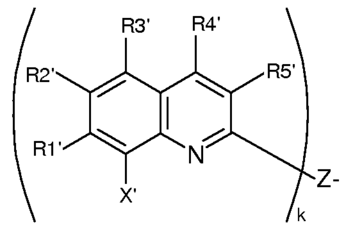

(IY)

in which X' represents an -NRR' group, and
Z represents -NR"-,
where R" = alkyl optionally substituted by one or more groups selected from OR, NRR',
and
R represents a hydrogen atom or an alkyl group ;
R'=Horalkyl;
R1, R2, R3, R4, R5, R1', R2', R3', R4' and R5' are the same or different, and independently represent an atom selected from H or Halogen,
k is 1 ;

as well as the pharmaceutically acceptable stereoisomers or mixtures, tautomeric forms, hydrates, solvates, salts, free forms and esters thereof.

**2.** A compound according to claim 1 selected from :

N-butyl-2,2'-imino-bis(8-quinoline amine)
as well as the pharmaceutically acceptable stereoisomers or mixtures, tautomeric forms, hydrates, solvates, salts, free forms and esters thereof.

**3.** A pharmaceutical composition comprising a compound of formula (I)

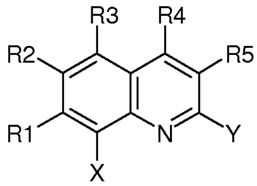

(I)

as defined in claim 1 or 2

**4.** Use of a compound of formula (I) as defined in claim 1 or 2 for the preparation of a medicament for preventing and / or treating diseases of the central nervous system, such as neurodegenerative diseases.

**5.** Use according to claim 4, such that the neurodegenerative diseases are selected from Alzheimer's disease, Parkinson's disease, Huntington's disease, amyotrophic lateral sclerosis, or trisomy 21, spongiform encephalopathies, such as Creutzfeldt-Jakob disease.

**6.** Use of a compound as defined according to claim 1 or 2 for the preparation of a medicament to be used for chelating metal ions and / or for dissolving amyloid aggregates.

**7.** Use according to claim 6 such that the said metals are selected from copper and / or iron.

**8.** A method for preparing a compound according to any one of claims 1 to 2 comprising the step of coupling the compounds of formula (VVII) and (VVII') :

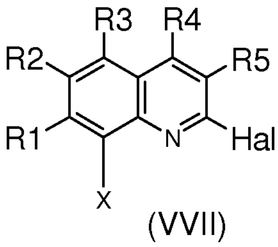

(VVII)

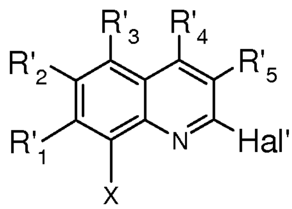

(VVII')

in which Hal and Hal' are halogen atoms in the presence of a group of the formula - RNH2, optionally followed by derivatisation(s) of the product of formula (I) obtained, in order to obtain the desired product of formula (I), and / or the step of isolation of the final product obtained.

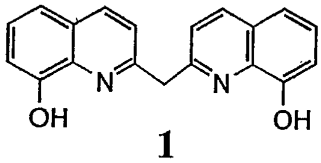

1

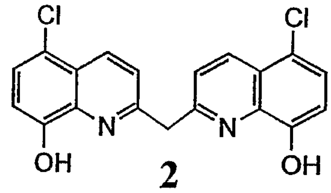

2

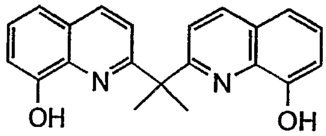

3

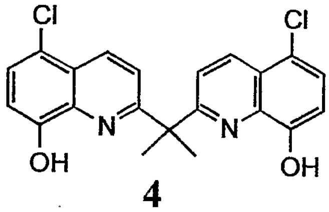

4

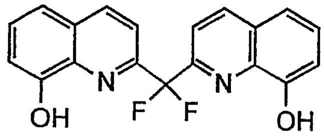

5

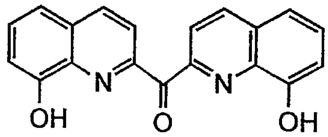

6

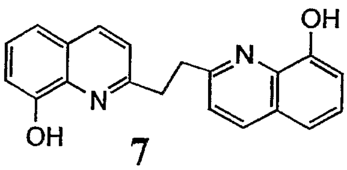

7

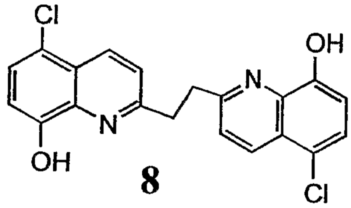

8

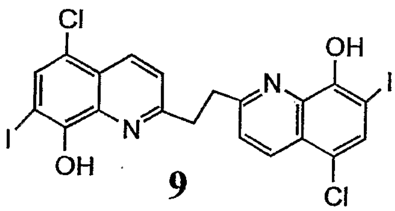

9

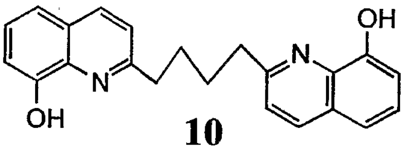

10

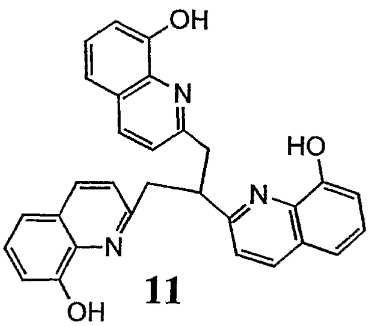

11

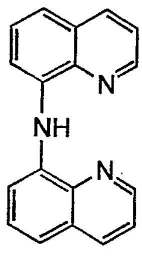

12

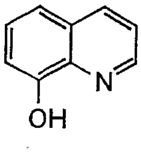

8-hydroxyquinoléine

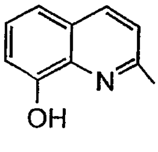

8-hydroxyqunaldine

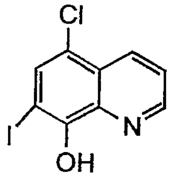

Clioquinol

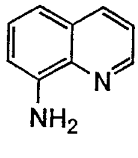

8-aminoquinoléine

FIG.1 (début)

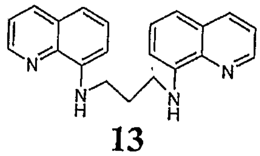

**13**

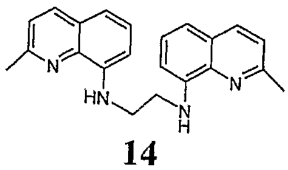

**14**

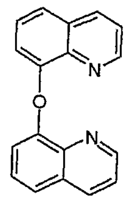

**15**

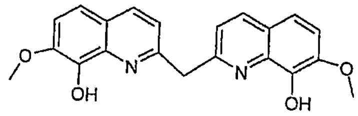

**16**

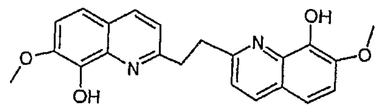

**17**

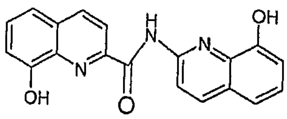

**18**

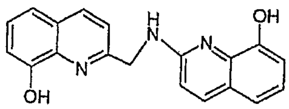

**19**

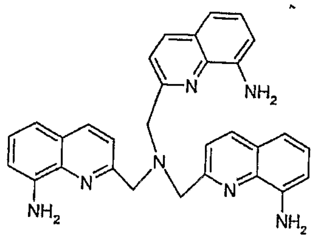

**21**

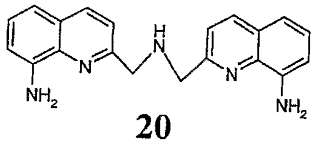

**20**

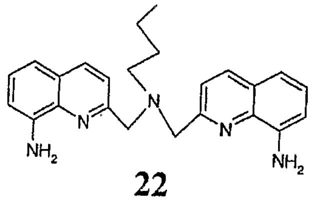

**22**

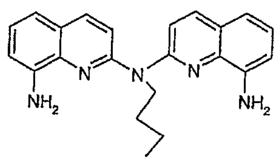

**23**

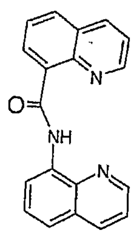

**24**

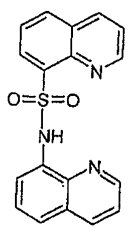

**25**

FIG.1 (fin)

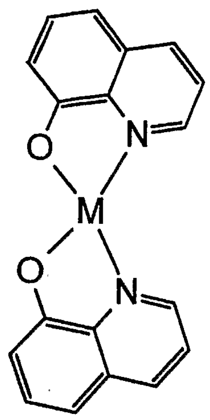

cis

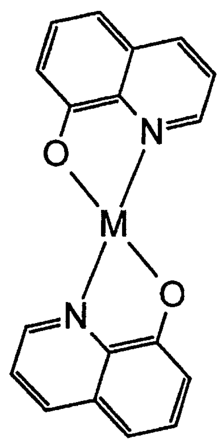

trans

FIG.2

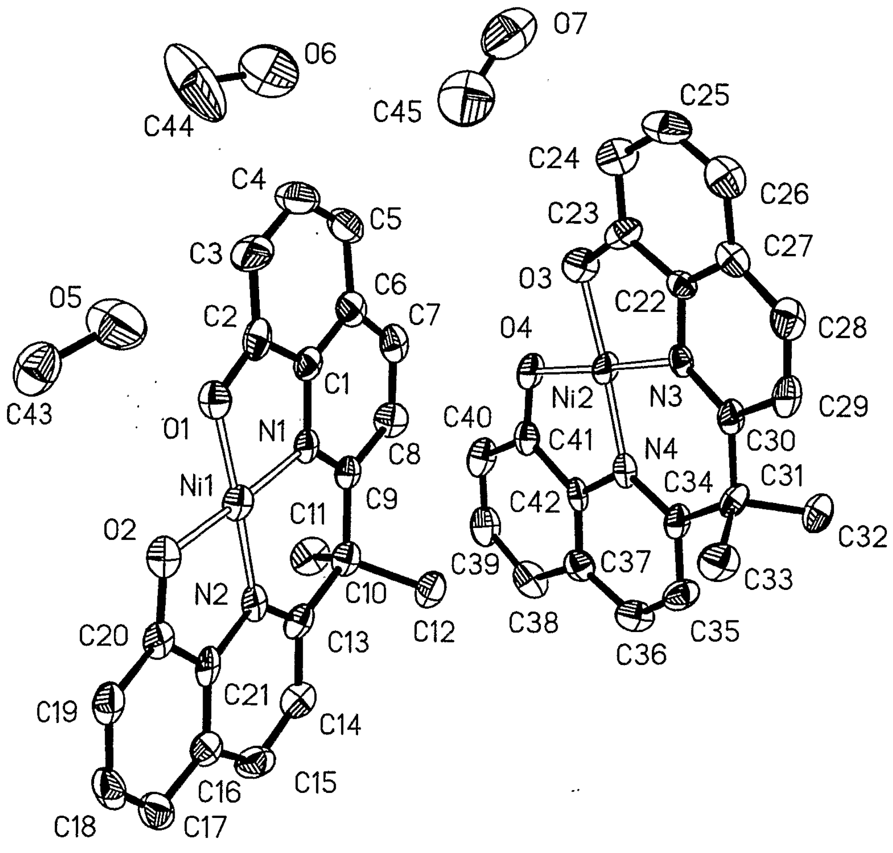

FIG.3

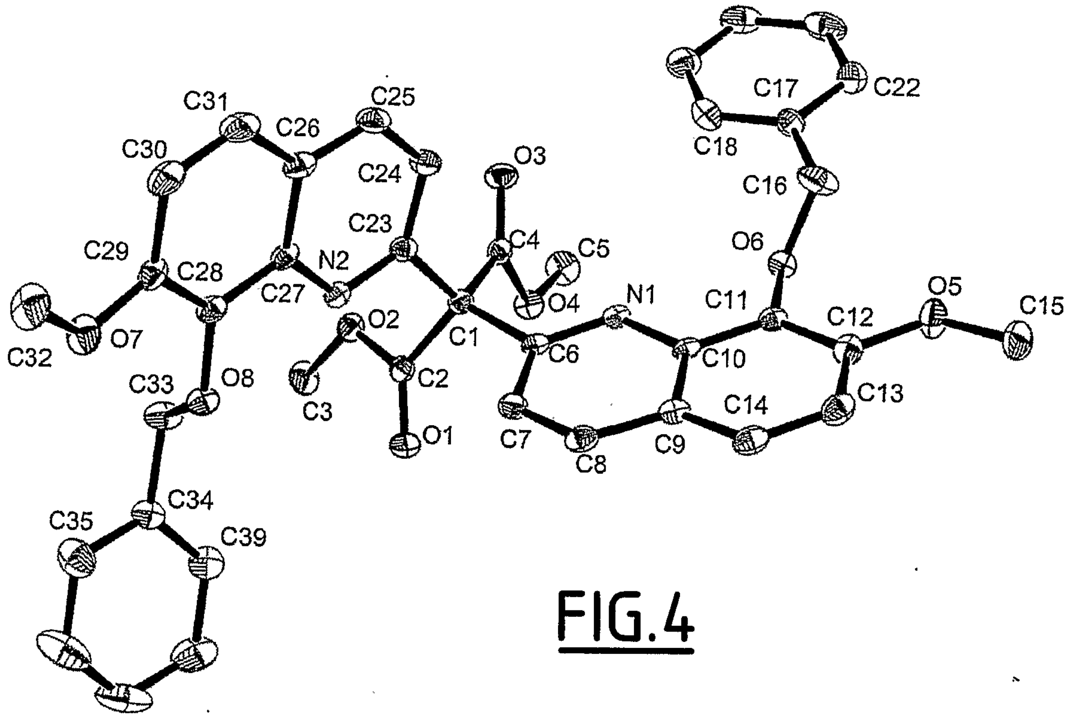

FIG.4

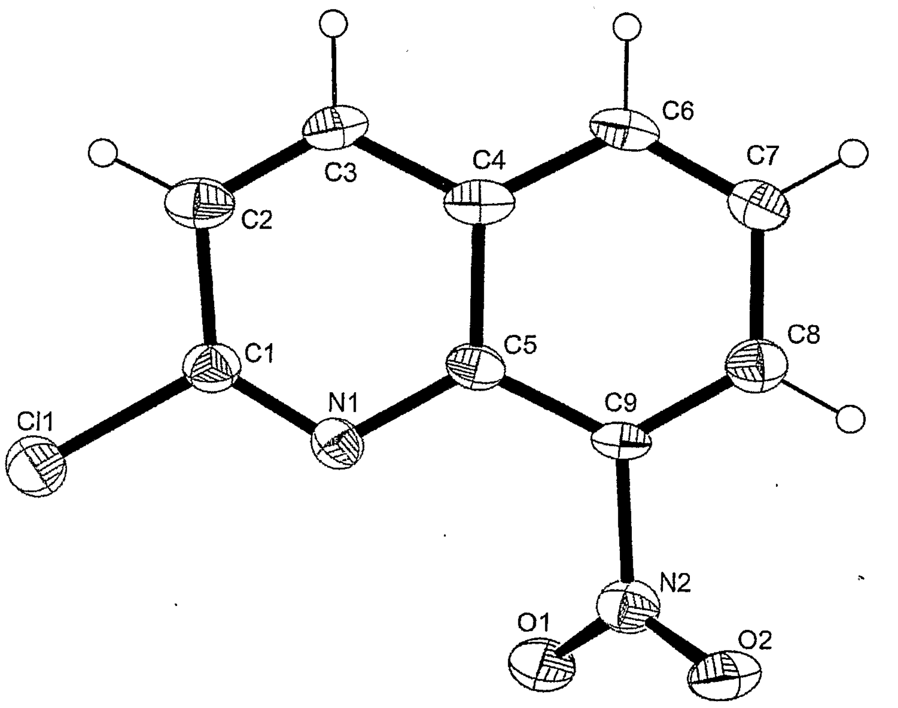

FIG.5

FIG.6

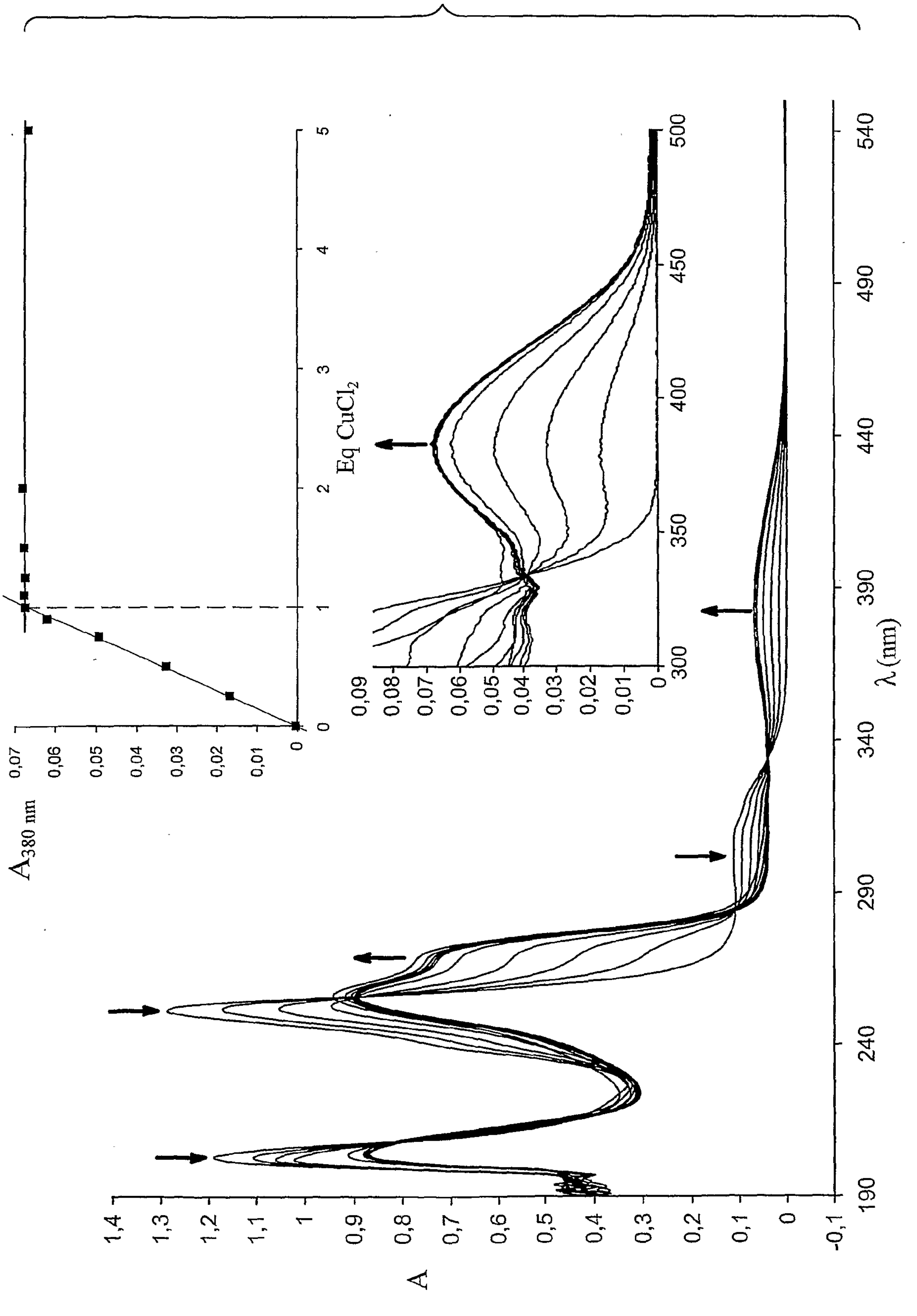

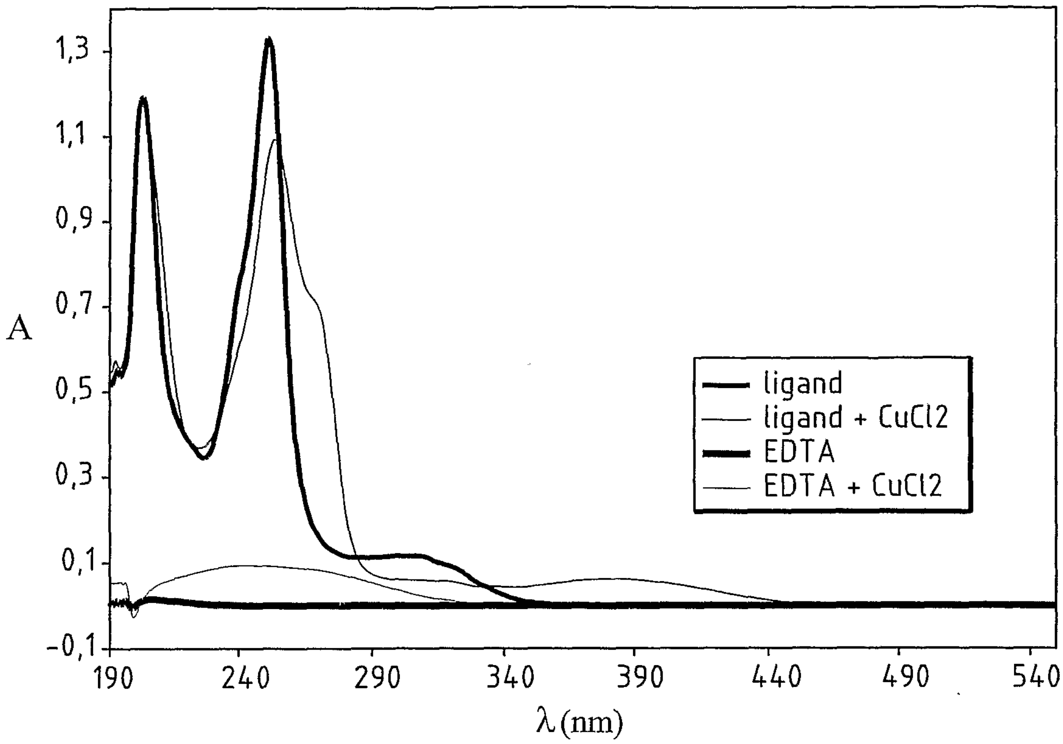
1,3
1,1
0,9
0,7
0,5
0,3
0,1
-0,1
A
ligand
ligand + CuCl2
EDTA
EDTA + CuCl2
190
240
290
340
390
440
490
540
λ (nm)

FIG.7

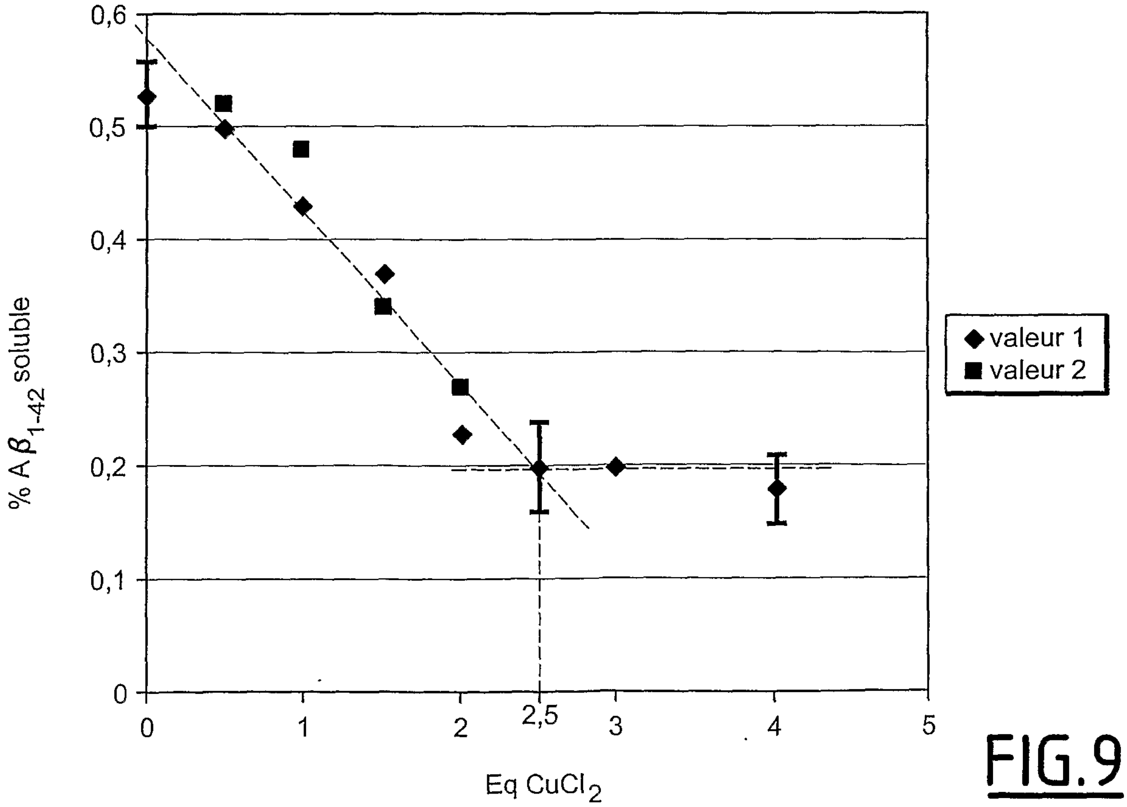
0,6
0,5
0,4
0,3
0,2
0,1
0
% A β1-42 soluble
valeur 1
valeur 2
0
1
2
2,5
3
4
5
Eq CuCl2

FIG.9

FIG.8

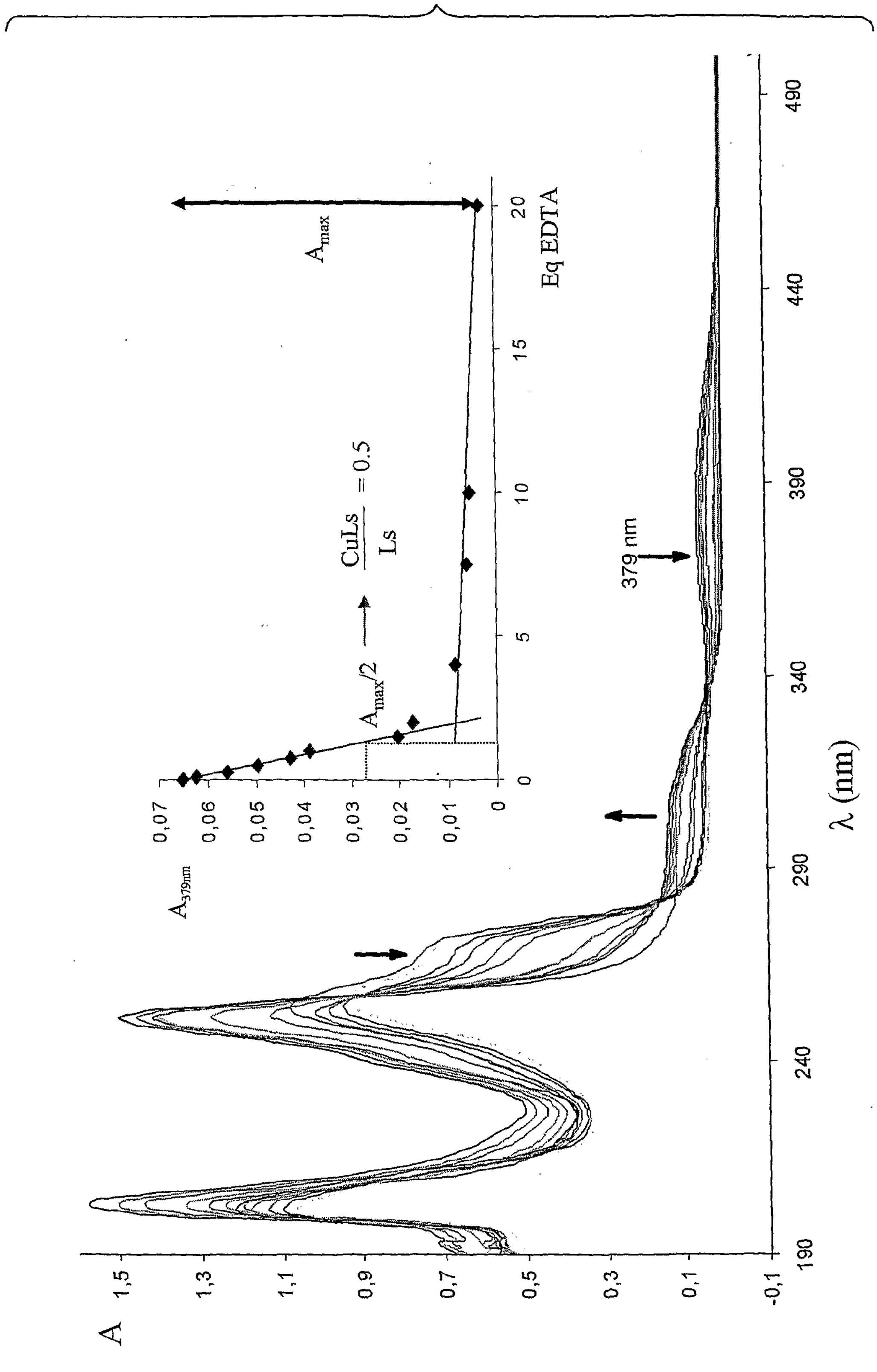
A
1,5
1,3
1,1
0,9
0,7
0,5
0,3
0,1
-0,1
190
240
290
340
390
440
490
λ (nm)
379 nm
$A_{379nm}$
0,07
0,06
0,05
0,04
0,03
0,02
0,01
0
0
5
10
15
20
Eq EDTA
$A_{max}$
$A_{max}/2$
$\frac{CuLs}{Ls} = 0.5$

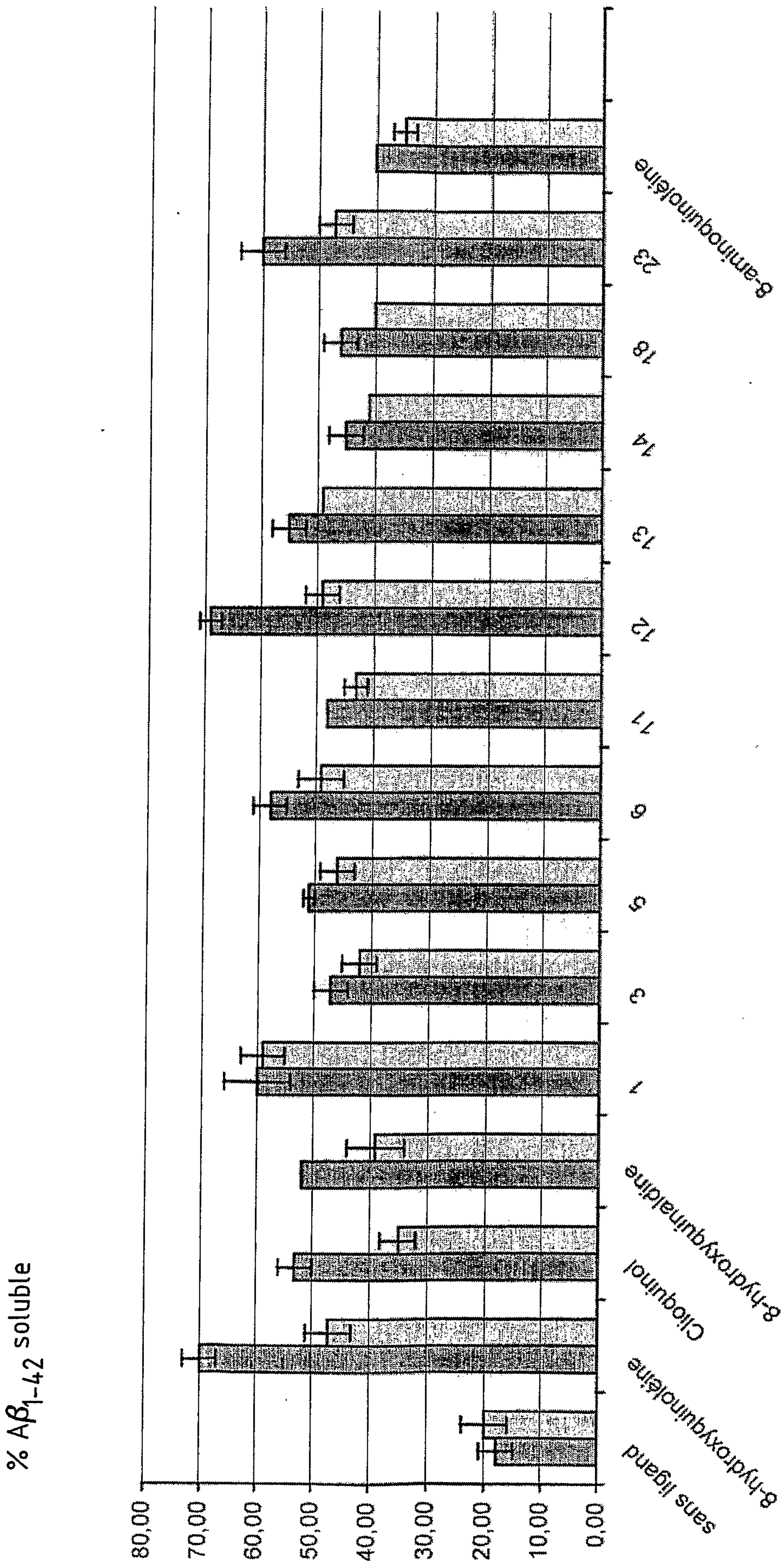
% Aβ1-42 soluble
80,00
70,00
60,00
50,00
40,00
30,00
20,00
10,00
0,00
sans ligand
8-hydroxyquinoléine
Clioquinol
8-hydroxyquinaldine
7
3
5
9
11
12
13
14
18
23
8-aminoquinoléine

FIG.10

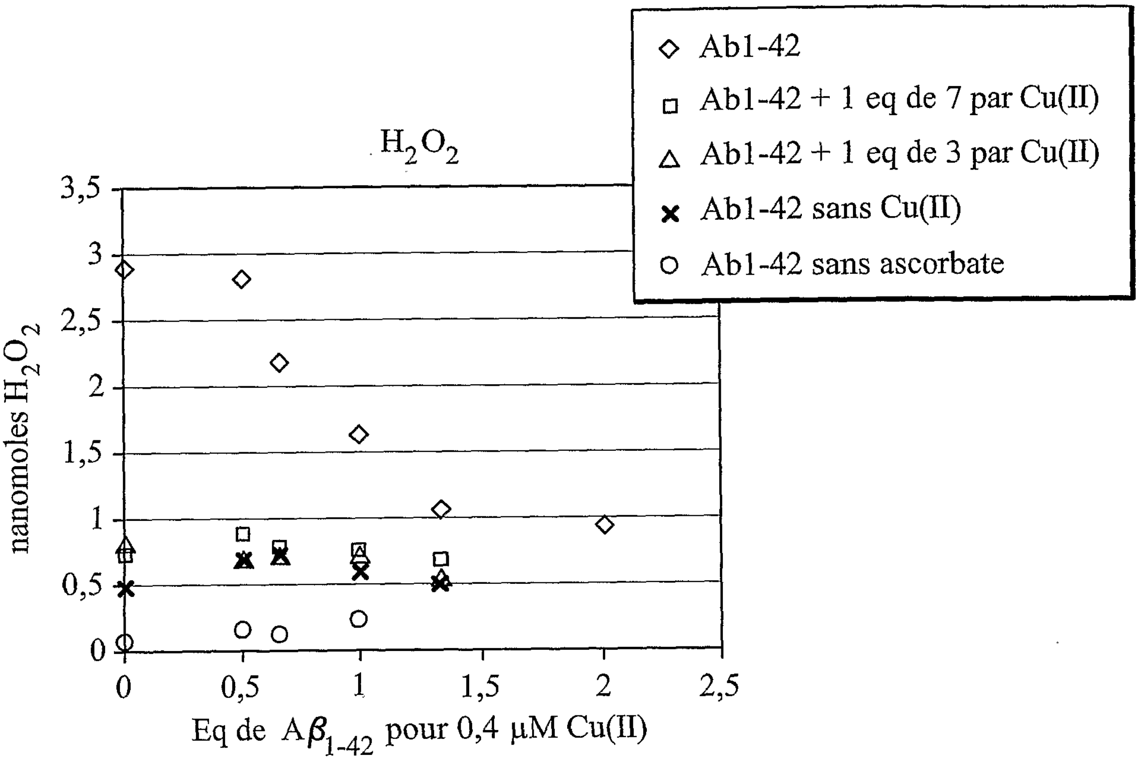
H2O2
Ab1-42
Ab1-42 + 1 eq de 7 par Cu(II)
Ab1-42 + 1 eq de 3 par Cu(II)
Ab1-42 sans Cu(II)
Ab1-42 sans ascorbate
nanomoles H2O2
3,5
3
2,5
2
1,5
1
0,5
0
0
0,5
1
1,5
2
2,5
Eq de Aβ1-42 pour 0,4 µM Cu(II)

FIG.11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004007461 A
- EP 0443862 A


### Littérature non-brevet citée dans la description


- **E. BOSSY-WETZEL et al.** *Nature Medecine,* 2004, S2-S9
- **K. J. BARNHAM et al.** *Nature Rev. Drug Discov.,* 2004, vol. 3, 205-214
- **M. P. MATTSON.** *Nature,* 2004, vol. 430, 631-639
- **P. M. DORAISWAMY et al.** *The Lancet Neurol.,* 2004, vol. 3, 431-434
- **E. QUAGLIO et al.** *J. Biol. Chem.,* vol. 276, 11432-11438
- **D. KAUR et al.** *Neuron,* 2003, vol. 37, 899-909
- **M. CITRON.** *Nature Rev. Neurosci.,* 2004, vol. 5, 677-685
- **M. P. CUAJUNGCO et al.** *Ann. N. Y. Acad. Sci.,* 2000, vol. 920, 292-304
- **C. S. ATWOOD et al.** *Met. Ions Biol. Syst.,* 1999, vol. 36, 309-364
- **R. A. CHERNY et al.** *Neuron,* 2001, vol. 30, 665-676
- **SILLEN, L. G. et al.** Stability Constants of Metal-Ion Complexes. The Chemical Society London Publication, 1971
- **STOCKWELL et al.** *J. Am. Chem. Soc.,* 1999, 10662-10663
- **E. SCARPINI et al.** *The Lancet Neurology,* 2003, vol. 2, 539-547
- **E. GAGGELI et al.** *Chem. Rev.,* 2006, vol. 106, 1995-2044
- **A. B. CLIPPINGDALE et al.** *J. Peptide Sc.,* 2001, vol. 7, 227-249
- **S.M. BERGE et al.** *Pharmaceutical Salts, J. Pharm. Sci,* 1977, vol. 66, 1-19
- Comprehensive Organic Transformations. VCH Pub, 1989
- **T.W. GREENE ; P.G.M. WUTS.** Protective Groups in Organic Chemistry. John Wiley and Sons, 1991
- **J.F.W. MCOMIE.** Protective Groups in Organic Chemistry. Plénum Press, 1973
- **C. KITAMURA et al.** *J. Chem. Soc., Perkin Trans. 1,* 2000, 781-785
- **H. JIANG et al.** *Tetrahedron,* 2003, vol. 59, 8365-8374
- **G. XUE et al.** *Tetrahedron,* 2001, vol. 57, 7623-7628
- **P. BELSER et al.** *Tetrahedron,* 1996, vol. 52, 2937-2944
- **D.E. PEARSON et al.** *J. Org. Chem.,* 1967, vol. 32, 2358-2360
- **G. E. COLLIS et al.** *Acta Cryst.,* 2003, vol. C59, o443-o444
- **M. C. KIMBER et al.** *Aust. J. Chem.,* 2003, vol. 56, 39-44
- **L. G. SILLEN ; A. E. MARTELL.** Stability Constants of Metai-Ion complexes. The Chemical Society London Publication, 1971
- **C. S. ATWOOD et al.** *J. Neurochem.,* 2000, vol. 75, 1219-1233
- **A. RINGBLOM.** Complexation in Analytical Chemistry. Interscience, 1963
- **G. SCHWARZNBACH et al.** Complexometric Titrations. Meuthuen, 1969
- **C. S. ATWOOD et al.** *J. Biol. Chem.,* 1998, vol. 273, 12817-12826
- **X. HUANG et al.** *J. Biol. Chem.,* 1999, vol. 274, 37111-37116
- **X. HUANG et al.** *Biochemistry,* 1999, vol. 38, 7609-7616
- **C. OPAZO et al.** *J. Biol. Chem.,* 2002, vol. 277, 40302-40308
- **K. J. BARNHAM et al.** *J. Biol. Chem.,* 2003, vol. 278, 42959-42965
- **G. D. CICCOTOSTO et al.** *J. Biol. Chem.,* 2004, vol. 279, 42528-42534
- **Z.-P ZHUANG et al.** *J. Med. Chem.,* 2001, vol. 44, 1905-1914
- **H. VAN DE WATERBEEMD et al.** *Nature Rev. Drug Discovery,* 2003, vol. 2, 192-204
- **D.M. MARON ; B. N. AMES.** *Mutat. Res.,* 1983, vol. 113, 173-215
- **D. E. LEVIN et al.** *Mutat. Res.,* 1982, vol. 94, 315-330
- **D. E. LEVIN et al.** *Proc. Natl. USA,* 1982, vol. 79, 7445-7449